# EUROPEAN PATENT SPECIFICATION

(11) **EP 2 620 433 B1**
(45) Date of publication and mention of the grant of the patent: **13.05.2015**
(21) Application number: 11826905.9
(22) Date of filing: 22.09.2011
(51) Int. Cl.: C07D 277/20, A61K 31/341, A61K 31/426, A61K 31/427, A61P 9/00, A61P 13/00, A61P 25/00, A61P 29/00, A61P 35/00, A61P 43/00, C07D 277/56, C07D 307/68, C07D 417/12

(54) **SUBSTITUTED AMIDE COMPOUND**
SUBSTITUIERTE AMIDVERBINDUNG
AMIDE SUBSTITUÉ

(30) Priority: 24.09.2010 WO PCT/JP2010/066572
(43) Date of publication of application: 31.07.2013
(73) Proprietor: Astellas Pharma Inc., Tokyo 103-8411 (JP)
(72) Inventor: KAWAMINAMI Eiji, Tokyo 103-8411 (JP); TAKAHASHI Tatsuhisa, Tokyo 103-8411 (JP); KANAYAMA Takatoshi, Tokyo 103-8411 (JP); SAKAMOTO Kazuyuki, Tokyo 103-8411 (JP)
(74) Representative: Hoffmann Eitle
(86) International application number: PCT/JP2011/071635
(87) International publication number: WO 2012/039460

(56) References cited:
- WO-A1-2004/031118
- WO-A1-2005/058790
- WO-A1-2010/051053
- WO-A1-2011/037192
- JP-A- 2009 523 774

## Description

### Technical Field

The present invention relates to a substituted amide compound which is useful as an active ingredient of a pharmaceutical composition, in particular, a pharmaceutical composition for preventing and/or treating diseases caused by lysophosphatidic acid (hereinafter abbreviated as LPA).

### Background Art

LPA is a phospholipid, for example, as represented by the following chemical formula, which has a simple structure containing a glycerol unit in which a fatty acid is present at the 1-position or 2-position and a phosphate group is bonded at the 3-position. Examples thereof include 1-acyl LPA, 1-alkyl LPA, 1-alkenyl LPA, 2-acyl LPA, and the like. Further, it has diversity depending on the type of the fatty acid, and may be classified into 18:1-LPA, 18:3-LPA, 16:0-LPA, and the like according to the length of the carbon chain and the degree of unsaturation.

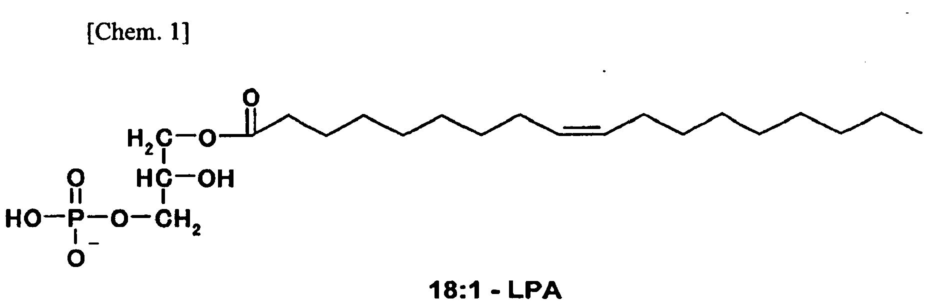

It is known that LPA is produced in various parts of the living body, both inside and outside of the cells, transduces signals into the cell mainly by binding to a G-protein coupled receptor present on the cell surface, and shows various physiological effects. 5 subtypes of LPA receptors are known, LPA1 to LPA5. Among these, three types of receptors, LPA1, LPA2, and LPA3 are also called EDG (Endothelial Differentiation Gene) 2, EDG4, and EDG7, respectively. The LPA receptors subtypes are distributed in various parts in the living body, but the localization tissue varies depending on the subtype, and it is thought that each receptor subtypes are involved in the biological functions of each tissue.

It has been reported that LPA is present in the semen in the lower urinary tract tissue (Non-Patent Document 1), and it has been revealed that LPA induces contraction of isolated urethral and prostate tissue strips in vitro, and increases the urethral pressure in vivo (Patent Document 1).

Furthermore, it has been reported that LPA induces contraction of isolated bladder smooth muscle cells, and LPA also promotes the proliferation of prostate cells obtained from benign prostatic hyperplasia (Non-Patent Documents 2 and 3).

In the nerve cells, LPA1 is highly expressed in oligodendrocytes and Schwann cells in a myelination period, and is expressed in correspondence with the period of myelination (Non-Patent Document 4).

It is also known that in a mouse model with demyelination, the amount of mRNA of LPA1 decreases by about 40% (Non-Patent Document 5).

It has been suggested that LPA inhibits the cell death of Schwann cells and oligodendrocytes, and is involved in the myelination (Non-Patent Document 6).

It has further been reported that LPA and LPA1 are involved in the expression of neuropathic pain (Non-Patent Document 7).

It has been shown that LPA is involved in various fibrotic diseases. It has been reported that in hepatic fibrosis, LPA promotes the contraction and proliferation of stellate cells which play an important role in the process of hepatic fibrosis and that the LPA concentration increases in patients with chronic hepatitis C and animal models with various hepatic diseases (Non-Patent Documents 8, 9, 10, and 11). It has further been reported that in renal fibrosis, the production of LPA and the expression of LPA1 increase in a mice with unilateral ureteral ligation model, which is an animal model of renal fibrosis, and the progression of fibrosis decreases in LPA1-deficient mice and LPA receptor antagonists (Non-Patent Document 12). It has been reported that with respect to pulmonary fibrosis, the LPA concentration in the bronchoalveolar lavage fluid in patients with idiopathic pulmonary fibrosis increases, that the LPA concentration in the bronchoalveolar lavage fluid increases in model mice with bleomycin-induced lung fibrosis, and that the progression of fibrosis and the death are remarkably inhibited in LPA1-deficient mice (Non-Patent Document 13).

In addition, it has been reported that LPA is accumulated to mediate the activation of platelets and endothelial cells by oxidized LDL in atherosclerosis lesions, and it has been suggested that LPA is involved in cardiovascular diseases (Non-Patent Document 14).

Furthermore, it is known that in the proliferative diseases, LPA promotes the migration of cancer cells (Non-Patent Document 15). It has been reported that the LPA concentration increases in the ascites of patients with ovarian cancer, and actually promotes the proliferation of the ovarian cancer cells (Non-Patent Documents 16 and 17). It has been reported that in prostate cancer, the expression of LPA1 receptor increases in the tumorlesion and the proliferation is enhanced in the prostate cancer cells overexpressing LPA1 (Non-Patent Document 18). It also has been reported that in breast cancer bone metastasis models, overexpression of LPA1 increases tumor proliferation/metastasi and LPA receptor antagonist inhibits the metastasis (Non-Patent Document 19). Further, in recent years, it has been rapidly revealed that various cells surrounding cancer cells assist the survival, growth, and distant metastasis of cancer cells in the cancer tissues. It has been revealed that human fat-derived mesenchymal stem cells differentiate into tumor-associated fibroblasts through the activation of LPA1 in tumor tissues by transplantation with cancer cells, thereby promoting the growth/angiogenesis of tumors (Non-Patent Document 20).

From the findings obtained by various studies on the LPA and LPA receptors, it is thought that an agent which inhibits the physiological activity of LPA, in particular, an antagonist of LPA1, may be useful as a drug for preventing or treating urologic diseases such as urinary disfunctionassociated with benign prostatic hyperplasia and the like, central/peripheral nervous system neurological diseases and uriological nerve diseases, hepatitis and renal insufficiency, fibrotic diseases such as idiopathic pulmonary fibrosis and the like, cardiovascular diseases such as atherosclerosis and the like, and proliferative diseases such as prostate cancer, breast cancer, ovarian cancer, and the like.

Meanwhile, it is known that a carboxylic acid derivative represented by the formula (A) has an LPA receptor antagonistic action and is useful for various diseases, for example, urinary system diseases, cancer-related diseases, proliferative diseases, inflammatory immune disease, brain-related diseases, chronic diseases, and the like (Patent Document 2). 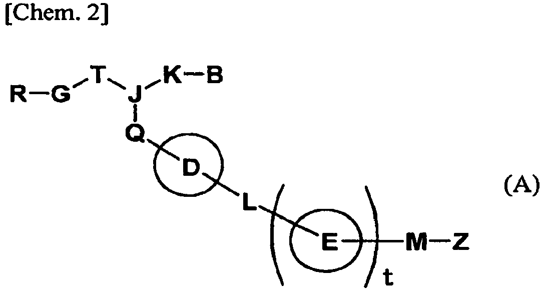 (wherein Z represents an acidic group, for the others, refer to the publication.)

It is further known that a compound represented by the formula (B) has an LPA receptor antagonistic action and is useful for various diseases, for example, urinary system diseases (symptoms associated with benign prostatic hyperplasia, neurogenic bladder diseases, and the like), cancer-related diseases, proliferative diseases, inflammatory immune diseases, brain-related diseases, chronic diseases, and the like (Patent Document 3). 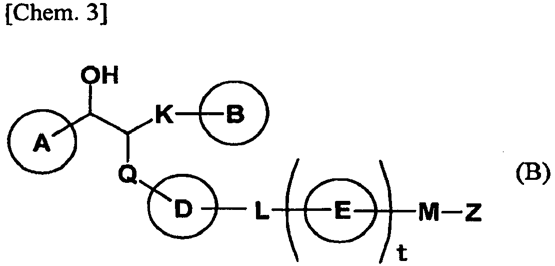 (for the symbols in the formula, refer to the publication.)

In any of the documents above, there is no specific disclosure of the compound of the present invention.

### Prior Art Document

### Non-Patent Document

[Non-Patent Document 1] FEBS Lett. 2002, 523, 187.
[Non-Patent Document 2] J. Urol. 1999, 162, 1779.
[Non-Patent Document 3] J. Urol. 2000, 163, 1027.
[Non-Patent Document 4] Eur. J. Neurosci. 1998, 10, 1045.
[Non-Patent Document 5] J. Comp. Neurol. 1998, 398, 587.
[Non-Patent Document 6] Proc. Natl. Acad. Sci. U.S.A. 1999, 96, 5233.
[Non-Patent Document 7] Nat. Med. 2004, 10, 712.
[Non-Patent Document 8] Biochem. Biophys. Res. Commun. 2000, 277, 72.
[Non-Patent Document 9] Biochem. Biophys. Res. Commun. 2000, 248, 436.
[Non-Patent Document 10] J. Clin. Gastroenterol. 2007, 41, 616.
[Non-Patent Document 11] Life Sci. 2008, 81, 1009.
[Non-Patent Document 12] J. Am. Soc. Nephrol. 2007, 18, 3110.
[Non-Patent Document 13] Nat. Med. 2007, 14, 45.
[Non-Patent Document 14] Proc. Natl Acad. Sci. U.S.A. 1999, 96, 6931.
[Non-Patent Document 15] Biochem. Biophysic. Res. Communic. 1993, 193, 497.
[Non-Patent Document 16] JAMA 1998, 280, 719.
[Non-Patent Document 17] J. Natl. Cancer. Inst. 2001, 93, 762.
[Non-Patent Document 18] Endocrinology 2006, 147, 4883.
[Non-Patent Document 19] Proc Natl Acad Sci U. S. A. 2006, 103, 9643.
[Non-Patent Document 20] Biochim Biophys Acta. 2010, 1801, 1205.
[Patent Document 1] Pamphlet of International Publication WO 02/062389
[Patent Document 2] Pamphlet of International Publication WO 2004/031118
[Patent Document 3] Pamphlet of International Publication WO 2005/058790

### Summary of Invention

### Problems to Be Solved by the Invention

The present invention provides a substituted amide compound which is useful as an active component of a pharmaceutical composition, in particular, a pharmaceutical composition for preventing and/or treating diseases caused by LPA.

### Means for Solving the Problems

The present inventors have made intensive studies on a compound having an antagonistic action against LPA receptor, and as a result, they have found that a substituted amide compound which is the compound of the present invention has an excellent antagonistic action against LPA receptor and is useful as an agent for preventing and/or treating diseases caused by LPA, thereby completing the present invention.

The present invention relates to the compound
2-({[(2-cyano-4-methoxyphenyl)acetyl](3-phenylpropyl)amino}methyl)-N-(dimethylsulfamoyl)-5-methyl-1,3-thiazole-4-carboxamide,
or a salt thereof.

The present invention relates to a pharmaceutical composition comprising 2-({[(2-cyano-4-methoxyphenyl)acetyl](3-phenylpropyl)amino}methyl)-N-(dimethylsulfamoyl)-5-methyl-1,3-thiazole-4-carboxamide or a salt thereof, and an excipient.

Furthermore, the present invention relates to pharmaceutical composition, in particular, a pharmaceutical composition for preventing and/or treating diseases caused by LPA, which comprises 2-({[(2-cyano-4-methoxyphenyl)acetyl](3-phenylpropyl)amino}methyl)-N-(dimethylsulfarnoyl)-5-methyl-1,3-thiazole-4-carboxamide or a salt thereof, and an excipient.

In addition, the present invention relates to the compound 2-({[(2-cyano-4-methoxyphenyl)acetyl](3-phenylpropyl)amino}methyl)-N-(dimethylsulfamoyl)-5-methyl-1,3-thiazole-4-carboxamide or a salt thereof for use in a method for preventing and/or treating diseases caused by LPA, comprising administering to a patient an effective amount of the compound or a salt thereof.

### Effects of the Invention

The compound 2-({[(2-cyano-4-methoxyphenyl)acetyl](3-phenylpropyl)amino}methyl)-N-(dimethylsulfamoyl)-5-methyl-1,3-thiazole-4-carboxamide or a salt thereof has an antagonistic action against LPA receptor and can be used as an agent for preventing and/or treating diseases caused by LPA.

### Best Mode for Carrying Out the Invention

Hereinafter, the present invention will be described in detail. Further, "the compound 2-({[(2-cyano-4-methoxyphenyl)acetyl](3-phenylpropyl)amino}methyl)-N-(dimethylsulfamoyl)-5-methyl-1,3-thiazole-4-carboxamide or a salt thereof" may be hereinafter denoted as "the compound of the present invention" or "the compound" in some cases.

In the present specification, the "diseases caused by LPA" refers to, for example, diseases such as urinary system diseases (benign prostatic hyperplasia (urinary disfunction associated with benign prostatic hyperplasia, and the like), overactive bladder, neurogenic bladder, bladder neck sclerosis, underactive bladder, and the like), central/peripheral neuropathy (neurogenic pain, painful peripheral diabetic neuropathy, nerve cell degeneration/nerve cell death after stroke, and the like), cancer-related diseases (prostate cancer, breast cancer, ovarian cancer, lung cancer, colon cancer, and the like), inflammatory diseases (rheumatoid arthritis, knee osteoarthritis, hepatitis C, and non-alcoholic steatohepatitis), diseases associated with fibrosis (chronic renal diseases, idiopathic pulmonary fibrosis, and chronic rejection after non-organ transplantation), cardiovascular diseases such as arteriosclerosis and the like. In another embodiment, examples of the diseases caused by LPA include urinary system diseases (benign prostatic hyperplasia (urinary disfunction associated with benign prostatic hyperplasia, and the like), overactive bladder, neurogenic bladder, bladder neck sclerosis, underactive bladder, and the like).

The compound may exist in the form of tautomers or geometrical isomers depending on the kind of substituents. In the present specification, the compound of the present invention shall be described in only one form of isomer, yet the present invention includes other isomers, isolated forms of the isomers, or a mixture thereof.

In addition, the compound of the present invention may have asymmetric carbon atoms or axial asymmetry in some cases, and correspondingly, it may exist in the form of optical isomers based thereon. The present invention includes both an isolated form of the optical isomers of the compound of the present invention or a mixture thereof.

Moreover, the present invention also includes a pharmaceutically acceptable prodrug of the compound of the present invention. The pharmaceutically acceptable prodrug is a compound having a group that can be converted into an amino group, a hydroxyl group, a carboxyl group, or the like through solvolysis or under physiological conditions. Examples of the group forming the prodrug include the groups described in Prog. Med., 5, 2157-2161 (1985) and "Pharmaceutical Research and Development" (Hirokawa Publishing Company, 1990), Vol. 7, Drug Design, 163-198.

Furthermore, the salt of the compound of the present invention is a pharmaceutically acceptable salt of the compound of the present invention and may form an acid addition salt or a salt with a base depending on the kind of substituents. Specific examples thereof include acid addition salts with inorganic acids such as hydrochloric acid, hydrobromic acid, hydroiodic acid, sulfuric acid, nitric acid, phosphoric acid, and the like, and with organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditolyltartaric acid, citric acid, methanesulfonic acid, ethanesulfonic acid, benzenesulfonic acid, p-toluenesulfonic acid, aspartic acid, glutamic acid, and the like, and salts with inorganic bases such as sodium, potassium, magnesium, calcium, aluminum, and the like or organic bases such as methylamine, ethylamine, ethanolamine, lysine, ornithine, and the like, salts with various amino acids or amino acid derivatives such as acetylleucine and the like, ammonium salts, etc.

In addition, the present invention also includes various hydrates or solvates, and polymorphic crystalline substances of the compound of the present invention and a salt thereof. In addition, the present invention also includes compounds labeled with various radioactive or non-radioactive isotopes.

### (Preparation Methods)

The compound of the present invention and a salt thereof can be prepared using the characteristics based on the basic structure or the type of substituents thereof and by applying various known synthesis methods. During the preparation, replacing the relevant functional group with a suitable protective group (a group that can be easily converted into the relevant functional group) at the stage from starting material to an intermediate may be effective depending on the type of the functional group in the production technology in some cases. The protective group for such a functional group may include, for example, the protective groups described in "Greene's Protective Groups in Organic Synthesis (4th edition, 2006)", P. G. M. Wuts and T. W. Greene, and one of these may be selected and used as necessary depending on the reaction conditions. In this kind of method, a desired compound can be obtained by introducing the protective group, by carrying out the reaction and by eliminating the protective group as necessary.

In addition, the prodrug of the compound of the present invention can be prepared by introducing a specific group or by carrying out the reaction using the obtained compound of the present invention at the stage from a starting material to an intermediate, just as in the case of the above-mentioned protective group. The reaction can be carried out using methods known to those skilled in the art, such as ordinary esterification, amidation, dehydration, and the like.

The compound can be isolated and purified as their free compounds, salts, hydrates, solvates, or polymorphic crystalline substances thereof. The salts of the compound of the present invention can be prepared by carrying out the treatment of a conventional salt forming reaction.

Isolation and purification are carried out by employing ordinary chemical operations such as extraction, fractional crystallization, various types of fractional chromatography, and the like.

Various isomers can be prepared by selecting an appropriate starting compound or separated by using the difference in the physicochemical properties between the isomers. For example, the optical isomers can be obtained by means of a general method for designing optical resolution of racemic products (for example, fractional crystallization for inducing diastereomer salts with optically active bases or acids, chromatography using a chiral column or the like, and others), and further, the isomers can also be prepared from an appropriate optically active starting compound.

The pharmacological activity of the compound of the present invention was confirmed by the tests shown below.

### Test Example 1 Antagonistic Action of the Compound of the Present Invention on Human LPA1

The antagonistic action on a human LPA1 was evaluated with an index of an inhibitory action on the LPA-stimulated increase in intracellular calcium ion concentration, using human LPA1-CHO cells [cells in which human LPA1 receptors are stably expressed in CHO (dhfr gene-deficient) cell lines].

Establishment of human LPA1-CHO cells was performed based on the basic genetic engineering techniques.

The established cells were maintained by passage in a nucleic acid-free α-MEM medium (Invitrogen) containing 10% FBS, 1% penicillin/streptomycin (Invitrogen), and 100 nM methotrexate, and before experiment, the medium was replaced with a medium that had been reduced to 1% of the PBS concentration, then seeded in 96-well plates to 1.5x10e⁵ cells/100 µL/well, and incubated overnight.

On the day of experiment, a 0.5 µM Fluo-4 solution [a solution preparedd by adding 20 mM HEPES (Sigma), 250 mM probenecid (Nacalai Tesque), 0.05% BSA, 0.5 µM Fluo-4 AM (Dojindo Laboratories), and 0.1 % Pluronic F217 (Molecular Probe Co.) to a Hanks Balanced Solt Solution (Invitrogen)] was, and loaded Fluo-4 on the cells by incubating for 2 hours at room temperature.

After loading Fluo-4, the Fluo-4 solution was replaced with a reaction solution [a solution obtained by adding 20 mM HEPES, 250 mM probenecid, and 0.05% BSA to a Hanks Balanced Solt Solution], and then measurement was performed using a device for measuring an intracellular calcium concentration (FLIPR tetra, Molecular Devices Inc.).

A reaction solution in which the compound of the present invention (with a final concentration of 0.1 nM to 10 µM) had been dissolved was added to each of the wells, the signals were measured over time for 4 minutes, then a reaction solution in which LPA (final concentration 100 nM) had been dissolved was added thereto, and the signals were measured over time for 2 minutes. The difference between the maximum and minimum response during one minute from addition of LPA was calculated. The inhibitory activity was calculated, with a response when LPA only (not including the compound) was added was taken as 0% inhibition, and a response when a reaction solution not including both of the compound and LPA was added was taken as 100% inhibition. Then the 50% inhibitory concentration was calculated as an IC₅₀ value (nM). The results are shown in Table 1. Example No. 1 - 18, and 20 - 28 are not part of the present invention and are provided for information purposes only.

Human LPA1-CHO cells used in the present test were the cells with the same sequence as the sequence described in Sequence NO.1 in the pamphlet of International Publication WO99/19513 or as the sequence described in Biochemical and Biophysical Research Communications, 1997, No. 231, pages 619-622. Further, Ex represents Example No. as denoted below.

**[Table 1]**

| Ex | IC₅₀ (nM) | Ex | IC₅₀ (nM) |
|---|---|---|---|
| 1 | 24 | 15 | 14 |
| 2 | 32 | 16 | 32 |
| 3 | 14 | 17 | 16 |
| 4 | 33 | 18 | 22 |
| 5 | 25 | 19 | 33 |
| 6 | 20 | 20 | 31 |
| 7 | 23 | 21 | 32 |
| 8 | 21 | 22 | 16 |
| 9 | 23 | 23 | 26 |
| 10 | 19 | 24 | 12 |
| 11 | 40 | 25 | 21 |
| 12 | 16 | 26 | 20 |
| 13 | 21 | 27 | 11 |
| 14 | 32 | 28 | 23 |

### Test Example 2 Inhibitory Action of Compound of the present invention on LPA-Induced Increase in Urethral Pressure in Rats under Anesthesia (with Intravenous Administration at 0.1 mg/kg)

Male Wistar rats (Charles River, 9- to 12-week old) are anesthetized with urethane (1.2 g/kg ip), and held in the supine position on an operating table kept at 37°C. The lower abdominal portion is midline-incised and the bladder is thus exposed. A small portion of the bladder apex is incised, a microchip pressure transducer (Millar) is inserted antegrade, and then placed in the urethra, and the urethral pressure is recorded continuously. In addition, a cannula for administration of a drug is placed into the femoral vein. After about 1 hour of stabilization, the compound of the present invention (0.1 mg/kg) is administered intravenously. After 5 minutes, LPA (1-oleoyl) is administered intravenously at 3 mg/kg, and the changes in the urethral pressure are recorded. The inhibitory rates (%) of the compound of the present invention on the LPA-stimulated increase in the urethral pressure compared with those after administration of the solvent of the compound of the present invention are recorded.

### Test Example 3 Estimation of Concentration in Plasma (2 Hours after Oral Administration) after Administration of Compound of the present invention in Rats Using Ex Vivo Bioassay Method

The concentration in the plasma after administration of the compound of the present invention in rats can be estimated according to a bioassay method. That is, test compounds are orally administered to male Wistar rats (Charles River, 6-week old, and fasted), and after a certain period of time, blood is collected from the ophthalmic basilar plexus to give plasma. The compound is extracted from the plasma, and the extracted compound is dissolved in a certain amount of DMSO. Further, for the standard curve, the plasma in which the compounds at various concentrations has been dissolved is prepared separately, and the same extraction procedure is conducted.

The inhibitory action on the LPA-stimulated increase in the intracellular calcium ion concentration in LPA1-expressing cells in the DMSO extract is measured, and the plasma concentration in the individual after administration is estimated from the standard curve.

### Text Example 4 Pharmacokinetic (PK) Test of Compound of the present invention in Body of Rat

As experimental animals, male SD rats which were fasted from the previous day of administration to 6 hours after administration and restricted from water from immediately before administration to 6 hours after administration were used.

The test substance, which had been dissolved in a solubilizing solution containing N,N-dimethylformamide, polyethylene glycol, or the like, was administered. In the case of oral administration, the test substance was forcibly orally administered (1 mg/5 mL/kg) using an oral sonde, while in the case of intravenous administration, the test substance was administered (1 mg/l mL/kg) to the caudal vein, or the total jugular vein or femoral vein that had been subjected to cannulation in advance. At a predetermined point of time after administration, the whole blood was sampled at each point of time in an amount of up to 250 uL in the presence of an anticoagulant (heparin or the like). In the case of oral administration, blood sampling was carried out at points of time of 0.25, 0.5, 1, 2, 4, 6, and 24 hours after administration, while in the case of intravenous administration, blood sampling was carried out at points of time of 0.1, 0.25, 0.5, 1, 2, 4, 6, and 24 hours after administration. Blood sampling was carried out from the venous plexus on the orbital floor, and the femoral artery cannula or caudal vein that had been was applied in advance.

The sampled blood was stored under freezing and then subjected to a centrifugation operation to obtain plasma. The concentration of the test substance in the plasma thus obtained was calculated by the measurement with LC/MS. As a result, for example, in the oral administration at 1 mg/kg, the Cₘₐₓ (the peak concentration in plasma) of the compound of Example 19 was 108 (ng/ mL), the AUC (concentration in plasma - area under the time curve) was 782 (ng·h/ mL), and the BA (bioavailability) was 90%.

As a result of the test above, it was confirmed that the compound of the present invention has an excellent LPA receptor antagonistic action. It was also confirmed that the compound of the present invention have excellent oral absorbability. Therefore, the compound can be used for treatment of diseases caused by LPA, or the like.

A pharmaceutical composition containing the compound of the present invention or a salt thereof as an active ingredient can be prepared using excipients that are usually used in the art, that is, excipients for pharmaceutical preparation, carriers for pharmaceutical preparation, and the like according to the methods usually used.

Administration can be accomplished either by oral administration via tablets, pills, capsules, granules, powders, solutions, and the like, or parenteral administration, such as injections such as intraarticular, intravenous, and intramuscular injections, suppositories, ophthalmic solutions, eye ointments, transdermal liquid preparations, ointments, transdermal patches, transmucosal liquid preparations, transmucosal patches, inhalers, and the like.

The solid composition for use in the oral administration is used in the form of tablets, powders, granules, or the like. In such a solid composition, one or more active ingredient(s) are mixed with at least one inactive excipient. In a conventional method, the composition may contain inactive additives, such as a lubricant, a disintegrating agent, a stabilizer, or a solubilization assisting agent. If necessary, tablets or pills may be coated with sugar or a film of a gastric or enteric coating substance.

The liquid composition for oral administration contains pharmaceutically acceptable emulsions, solutions, suspensions, syrups, elixirs, or the like, and also contains generally used inert diluents, for example, purified water or ethanol. In addition to the inert diluent, the liquid composition may also contain auxiliary agents, such as a solubilization assisting agent, a moistening agent, and a suspending agent, sweeteners, flavors, aromatics, or antiseptics.

The injections for parenteral administration include sterile aqueous or non-aqueous solutions, suspensions and emulsions. The aqueous solvent includes, for example, distilled water for injection and physiological saline. Examples of the non-aqueous solvent include alcohols such as ethanol. Such a composition may further contain a tonicity agent, an antiseptic, a moistening agent, an emulsifying agent, a dispersing agent, a stabilizer, or a solubilizing aid. These are sterilized, for example, by filtration through a bacteria retaining filter, blending of a bactericide, or irradiation. In addition, these can also be used by preparing a sterile solid composition, and dissolving or suspending it in sterile water or a sterile solvent for injection prior to its use.

The agent for external use includes ointments, plasters, creams, jellies, poultices, sprays, lotions, eye drops, eye ointments, and the like. The agents contain generally used ointment bases, lotion bases, aqueous or non-aqueous liquid preparations, suspensions, emulsions, and the like.

As the transmucosal agents such as an inhaler, a transnasal agent, and the like, those in the form of a solid, liquid, or semi-solid state are used, and can be prepared in accordance with a conventionally known method. For example, a known excipient, and also a pH adjusting agent, an antiseptic, a surfactant, a lubricant, a stabilizer, a thickening agent, or the like may be appropriately added thereto. For their administration, an appropriate device for inhalation or blowing can be used. For example, a compound may be administered alone or as a powder of formulated mixture, or as a solution or suspension in combination with a pharmaceutically acceptable carrier, using a known device or sprayer, such as a measured administration inhalation device, and the like. A dry powder inhaler or the like may be for single or multiple administration use, and a dry powder or a powder-containing capsule may be used. Alternatively, this may be in a form such as a pressurized aerosol spray which uses an appropriate ejection agent, for example, a suitable gas such as chlorofluoroalkane, carbon dioxide, and the like.

In oral administration, the daily dose is generally from about 0.001 to 100 mg/kg, preferably from 0.1 to 30 mg/kg, and more preferably 0.1 to 10 mg/kg, per body weight, administered in one portion or in 2 to 4 divided portions. In the case of intravenous administration, the daily dose is suitably administered from about 0.0001 to 10 mg/kg per body weight, once a day or two or more times a day. In addition, a transmucosal agent is administered at a dose from about 0.001 to 100 mg/kg per body weight, once a day or two or more times a day. The dose is appropriately decided in response to the individual case by taking the symptoms, the age, and the gender, and the like into consideration.

The compound of the present invention can be used in combination with various therapeutic or prophylactic agents for the diseases for which the compound of the present invention is considered to be effective, as described above. The combined preparation may be administered simultaneously, or separately and continuously, or at a desired time interval. The preparations to be administered simultaneously may be a blend, or may be prepared individually.

### Examples

Hereinbelow, the preparation methods for the compound of the present invention will be described in more detail with reference to Examples. Further, the present invention is not limited to only the preparation methods of the specific Examples and Preparation Examples below, but the compound of the present invention can be prepared by a combination of the preparation methods or a method apparent to a person skilled in the art. Example No. 1 - 18, and 20 - 28 are not part of the present invention. Data relating to these Examples are provided for information purposes only.

Moreover, the following abbreviations may be used in some cases in the Examples, Preparation Examples, and Tables as described later.
Rf: Preparation Example No.,
Ex: Example No.,
Data: Physicochemical data,
ESI+: representing m/z values in ESI-MS (positive ions), and representing [M+H]⁺ peaks unless otherwise specified,
ESI-: representing m/z values in ESI-MS (negative ions), and representing [M-H]⁻ peaks unless otherwise specified,
APCI+: representing m/z values in APCI-MS (positive ions), and representing [M+H]⁺ peaks unless otherwise specified,
FAB+: representing m/z values in FAB-MS (positive ions), and representing [M+H]⁺ peaks unless otherwise specified,
FAB-: representing m/z values in FAB-MS (negative ions), and representing [M-H]⁻ peaks unless otherwise specified,
EI+: representing m/z values in EI-MS (positive ions), and representing [M]⁺ peaks unless otherwise specified,
NMR-DMSO-d₆: δ (ppm) in ¹H NMR in DMSO-d₆,
NMR-CDCl₃: δ (ppm) in ¹H NMR in CDCl₃,
Structure: Structural formula,
Syn: Preparation method (in which the numeral shows that the compound is prepared by the same way in the compound having the Example No. and R prefixed before the numeral shows that the compound is prepared in the same way as the compound having the Preparation Example No.),
HCl: hydrochloride,
brine: saturated brine,
DMSO: dimethylsulfoxide,
THF: tetrahydrofuran,
EtOH: ethanol,
DMF: N,N-dimethylformamide,
MeOH: methanol,
CHCl₃: chloroform,
CDI: 1,1'-carbonyldiimidazole,
DBU: 1,8-diazabicyclo[5.4.0]undec-7-ene,
DMAP: 4-dimethylaminopyridine,
NBS: N-bromosuccinimide,
AIBN: 2,2'-azobis(isobutyronitrile),
Pd (PPh₃)₄: tetrakis(triphenylphosphine) palladium (0),
Zn(CN)₂: dicyanozinc,
HATU: O-(7-azabenzotriazol-1-yl)-N,N,N',N'-tetramethyluronium hexafluorophosphate,
DBAD: di-tert-butylazodicarboxylate,
MgSO₄: anhydrous magnesium sulfate,
Na₂SO₄: anhydrous sodium sulfate,
M: mol/L.

### Preparation Example 1

1-(5-Methoxypyridin-2-yl)cyclopropanecarbonitrile (100 mg) and a 5 M aqueous potassium hydroxide solution (2 mL) were added to ethylene glycol (2 mL), followed by heating at 120°C overnight. To the reaction mixture was added an appropriate amount of ice water, and 1 M hydrochloric acid was further added thereto to adjust the mixture to be weakly acidic, followed by extraction with ethyl acetate. The organic layer was washed with brine, dried over Na₂SO₄, and then concentrated under reduced pressure to prepare 1-(5-methoxypyridin-2-yl)cyclopropanecarboxylic acid (55 mg).

### Preparation Example 2

2-(Chloromethyl)-5-methoxypyridine (125 mg) was added to DMSO (5 mL), and subsequently, an aqueous potassium cyanide solution (potassium cyanide (155 mg) and water (1 mL)) was added thereto, followed by stirring at room temperature overnight. An appropriate amount of purified water was poured into the reaction mixture under ice-cooling, followed by extraction with ethyl acetate. The obtained organic layer was sequentially washed with purified water and brine, and dried over MgSO_{4,} and then the solvent was evaporated under reduced pressure to prepare (5-methoxypyridin-2-yl)acetonitrile (110 mg).

### Preparation Example 3

To a mixture of (5-methoxypyridin-2-yl)acetonitrile (0.11 mg), 1-bromo-2-chloroethane (0.2 mL), and N-benzyl-N,N,N-triethylammonium chloride (20 mg) was slowly added dropwise a 50% aqueous sodium hydroxide solution (2 mL) under ice-cooling, followed by stirring at room temperature for about 5 hours. Ice water was poured into the reaction mixture, followed by extraction with diethyl ether. The organic layer was washed with brine, dried over Na₂SO₄, and then concentrated under reduced pressure. The obtained residue was purified by silica gel column chromatography (ethyl acetate:hexane=1:2) to prepare 1-(5-methoxypyridin-2-yl)cyclopropanecarbonitrile (100 mg) as a white solid.

### Preparation Example 4

Ethyl 2-methyl-1,3-thiazole-4-carboxylate (10 g) was added to acetonitrile (100 mL), and subsequently, NBS (11.4 g) was added thereto, followed by stirring for 3 hours under heating to reflux. To the reaction mixture was added NBS (5.0 g), followed by stirring for 2 hours under refluxing, and then NBS (5.0 g) was further added thereto, followed by stirring for about 12 hours under the same condition. An appropriate amount of a saturated aqueous sodium hydrogen carbonate solution was slowly poured into the reaction mixture under cooling, followed by extraction with ethyl acetate. The organic layer was washed with brine and dried over MgSO_{4,} and then the solvent was evaporated. The obtained residue was purified by silica gel column chromatography (hexane:ethyl acetate=3:2) to prepare ethyl 5-bromo-2-methyl-1,3-thiazole-4-carboxylate (8.86 g).

### Preparation Example 5

Ethyl 5-bromo-2-methyl-1,3-thiazole-4-carboxylate (6.84 g) was added to carbon tetrachloride (114 mL), and subsequently, NBS (5.35 g) and AIBN (2.25 g) were added thereto, followed by stirring at about 90°C for 2 hours, and then NBS (5.0 g) and AIBN (0.9 g) were added thereto, followed by heating to reflux for additional 1 hour. The reaction mixture was left to be cooled, then the insoluble material was collected by filtration, and the filtrate was concentrated under reduced pressure. The obtained residue was purified by silica gel column chromatography (hexane:ethyl acetate=3:2) to prepare ethyl 5-bromo-2-(bromomethyl)-1,3-thiazole-4-carboxylate (5.55 g).

### Preparation Example 6

Ethyl 2-methyl-1,3-thiazole-4-carboxylate (10 g) was added to DMF (100 mL) under ice-cooling, and subsequently, trichloroisocyanuric acid (13.6 g) was slowly added thereto, followed by stirring at room temperature overnight. Thereafter, an equivalent amount of trichloroisocyanuric acid was added thereto several times in divided portions, followed by stirring at room temperature for one day. The insoluble material in the reaction mixture was collected by filtration through Celite, and to the filtrate was added ice water including an appropriate amount of a 1 M aqueous sodium hydroxide solution, followed by extraction with ethyl acetate. The organic layer was washed with brine and dried over MgSO_{4,} and then the solvent was evaporated. The obtained residue was purified by silica gel column chromatography (hexane:ethyl acetate=7:3→1:1) to prepare ethyl 5-chloro-2-methyl-1,3-thiazole-4-carboxylate (6.7 g).

### Preparation Example 7

Ethyl 2-(diethoxymethyl)-5-methyl-1,3-thiazole-4-carboxylate (12.1 g) was added to acetone (300 mL), and subsequently, 1 M hydrochloric acid (150 mL) was added thereto, followed by stirring at 55°C for about 5 hours. The reaction mixture was concentrated, neutralized by the addition of an appropriate amount of a saturated aqueous sodium hydrogen carbonate solution, and then extracted with ethyl acetate several times. The organic layer was dried over MgSO_{4,} and the solvent was evaporated under reduced pressure to prepare ethyl 2-formyl-5-methyl-1,3-thiazole-4-carboxylate (8.25 g).

### Preparation Example 8

2,2-diethoxyethanethioamide (9.21 g), calcium carbonate (3.39 g), and an appropriate amount of powder Molecular Sieves (4 Angstrom, about 2 times a medicinal spoon) were added to EtOH (220 mL), and subsequently, ethyl 3-bromo-2-oxobutanoate (13.1 g) prepared by the method by Plouvier, et al. (Heterocycles, 1991 32, 693.) was added dropwise thereto over about 5 minutes, followed by stirring at room temperature for about 30 minutes. Thereafter, the mixture was further warmed to 55°C for about 6 hours. The reaction mixture was left to be cooled, then the insoluble material was removed by filtration, and the filtrate was concentrated under reduced pressure. To the obtained residue was added an appropriate amount of water, followed by extraction with ethyl acetate twice. The organic layer was washed with brine and dried over MgSO_{4,} and then the solvent was evaporated. The obtained residue was purified by silica gel column chromatography (hexane:ethyl acetate=7:3) to prepare ethyl 2-(diethoxymethyl)-5-methyl-1,3-thiazole-4-carboxylate (12.1 g).

### Preparation Example 9

3-Phenylpropan-1-amine (1.3 g) was added to methylene chloride (30 mL), and subsequently, ethyl 2-formyl-5-methyl-1,3-thiazole-4-carboxylate (1.2 g) and acetic acid (1.5 mL) were sequentially added thereto, followed by stirring at room temperature for about 20 minutes. Thereafter, sodium triacetoxyborohydride (2.69 g) was added thereto under ice-cooling, followed by stirring at room temperature for about 1 hour. To the reaction mixture was added CHCl₃, and an appropriate amount of a saturated aqueous sodium hydrogen carbonate solution was further added thereto, followed by stirring and then performing liquid-separation. The organic layer was dried over MgSO_{4,} and then the solvent was evaporated under reduced pressure. The obtained yellow oily substance was purified by silica gel column chromatography (CHCl₃:MeOH=250:1) to prepare ethyl 5-methyl-2-{[(3-phenylpropyl)amino]methyl}-1,3-thiazole-4-carboxylate (1.56 g).

### Preparation Example 10

2-Fluoro-4-methoxybenzaldehyde (1.0 g), triethylamine (0.2 mL), and trimethylsilylcyanide (0.9 mL) were added to methylene chloride (10 mL), followed by stirring at room temperature for 3 hours. The reaction mixture was concentrated under reduced pressure, and to the obtained residue were added EtOH (12 mL) and chlorotrimethylsilane (12 mL), followed by stirring at room temperature overnight. The reaction mixture was left to be cooled, and then the solvent was evaporated. Dichloroethane (20 mL), EtOH (10 mL), and a saturated aqueous sodium hydrogen carbonate solution (20 mL) were poured into the obtained residue, followed by severely stirring at room temperature for about 3 hours. The reaction mixture was extracted with an appropriate amount of CHCl₃, the organic layer was dried over MgSO_{4,} and then the solvent was evaporated under reduced pressure to prepare ethyl (2-fluoro-4-methoxyphenyl)(hydroxy)acetate (0.67 g).

Subsequently, (2-fluoro-4-methoxyphenyl)(hydroxy)acetic acid (0.35 g) was prepared from ethyl (2-fluoro-4-methoxyphenyl)(hydroxy)acetate (0.67 g) in the same manner as the method of Preparation Example 24.

### Preparation Example 11

1-(4-Hydroxyphenyl)cyclopropanecarboxylic acid (1.07 g) was added to EtOH (20 mL), and concentrated sulfuric acid (0.1 mL) was added dropwise thereto, followed by stirring at 70°C for 2 days. The solvent was evaporated under reduced pressure, and to the obtained residue was added a saturated aqueous sodium hydrogen carbonate solution, followed by extraction with an appropriate amount of ethyl acetate. The organic layer was washed with brine and dried over Na₂SO₄, and then the solvent was evaporated under reduced pressure. The obtained residue was purified by silica gel column chromatography (hexane:ethyl acetate=2:1) to prepare ethyl 1-(4-hydroxyphenyl)cyclopropanecarboxylate (1.15 g) as a pale yellow solid.

Subsequently, ethyl 1-(4-hydroxyphenyl)cyclopropanecarboxylate (200 mg), triphenylphosphine (382 mg), and 2-fluoroethanol (93 mg) were added to THF, and subsequently, DBAD (335 mg) was added thereto under ice-cooling, followed by stirring at room temperature overnight. The solvent was evaporated under reduced pressure, and the obtained residue was purified by silica gel column chromatography (hexane:ethyl acetate=2:1) to prepare ethyl 1-[4-(2-fluoroethoxy)phenyl]cyclopropanecarboxylate (190 mg) as a colorless oily substance.

Furthermore, ethyl 1-[4-(2-fluoroethoxy)phenyl]cyclopropanecarboxylate (190 mg) was added to an EtOH/THF (1:1) solution (10 mL), and subsequently, a 1 M aqueous sodium hydroxide solution (2 mL) was added dropwise thereto, followed by stirring at room temperature overnight. The reaction mixture was concentrated under reduced pressure and neutralized by the addition of purified water and 1 M hydrochloric acid, and then resulting insoluble material was collected by filtration to prepare 1-[4-(2-fluoroethoxy)phenyl]cyclopropanecarboxylic acid (152 mg) as a white solid.

### Preparation Example 12

The following products were prepared with a partial modification of the method of Johnson, et al. (Tetrahedron Lett., 2004 45, 8483-8487.).

N-Benzylmethane sufonamide (2.0 g) was added to THF (40 mL), and subsequently, a 1.66 M solution of n-butyl lithium in n-hexane (13.1 mL) was added dropwise thereto under cooling at -78°C, followed by stirring for 5 minutes and then warming to 0°C. To the reaction mixture was slowly added dropwise a mixture prepared by adding acetaldehyde (2.4 mL) to THF (20 mL), followed by stirring for 2 hours while warming to room temperature. To the reaction mixture was added an aqueous ammonium chloride solution, followed by extraction with CHCl₃, the aqueous layer was separated with a phase separator, and the solvent of the organic layer was evaporated. The obtained residue was purified by silica gel column chromatography (CHCl₃:MeOH=20:1) to prepare N-benzyl-2-hydroxypropane-1-sufonamide (1.94 g) as a white solid.

Subsequently, N-benzyl-2-hydroxypropane-1-sufonamide (1.94 g), DMAP (0.52 g), triethylamine (1.77 mL), and tert-butyldimethylchlorosilane (1.91 g) were added to methylene chloride (50 mL), followed by stirring at room temperature overnight. To the reaction mixture was added an aqueous ammonium chloride solution, followed by extraction with ethyl acetate. The organic layer was washed with brine and dried by the addition of MgSO₄, and the solvent was evaporated. The obtained residue was purified by silica gel column chromatography (hexane:ethyl acetate=2:1) to prepare N-benzyl-2-{[tert-butyl (dimethyl)silyl]oxy}propane-1-sufonamide (1.84 g).

Furthermore, N-benzyl-2- {[tert-butyl (dimethyl)silyl]oxy}propane-1-sufonamide (1.8 g) and 10% palladium hydroxide (0.5 g) were added to ethyl acetate (30 mL), followed by stirring at room temperature for 3 hours under a hydrogen atmosphere. The reaction mixture was filtered through Celite and the solvent was evaporated. The obtained residue was purified by silica gel column chromatography (CHCl₃:MeOH=10:1) to prepare 2-{[tert-butyl (dimethyl)silyl]oxy}propane-1-sufonamide (1.04 g) as a white solid.

### Preparation Example 13

tert-Butyl [(4-{[(dimethylamino)sulfonyl]carbamoyl}-5-methyl-1,3-thiazol-2-yl)methyl](3-phenylpropyl)carbamate (4.12 g) was added to dioxane (30 mL), and then a 4 M hydrochloric acid/dioxane solution (30 mL) was poured thereinto, followed by stirring at room temperature for about 12 hours under a sealed argon gas atmosphere. The reaction mixture was concentrated under reduced pressure to prepare a white solid of N-(dimethylsulfamoyl)-5-methyl-2-{[(3-phenylpropyl)amino]methyl}-1,3-thiazole-4-carboxamide hydrochloride (3.4 g).

### Preparation Example 17

2-{[(tert-Butoxycarbonyl)(3-phenylpropyl)amino]methyl}-5-methyl-1,3-thiazole-4-carboxylic acid (4.0 g) was added to anhydrous THF (120 mL), and then CDI (2.49 g) was added thereto, followed by stirring at about 60°C for 1.5 hours under an argon atmosphere. The reaction mixture was ice-cooled, and N,N-dimethylsulfamide (2.54 g) and DBU (2.34 g) were sequentially added thereto, followed by stirring at room temperature for about 13 hours. To the reaction mixture were added an appropriate amount of 1 M hydrochloric acid and ice water, followed by extraction with ethyl acetate several times. The organic layer was washed with brine and dried over MgSO_{4,} and the solvent was evaporated under reduced pressure. The obtained residue was purified by silica gel column chromatography (hexane:ethyl acetate=1:3) to prepare a colorless syrup of tert-butyl [(4-{[(dimethylamino)sulfonyl]carbamoyl}-5-methyl-1,3-thiazol-2-yl)methyl](3-phenylpropyl)carbamate (4.12 g).

### Preparation Example 19

Ethyl 5-methyl-2-{[(3-phenylpropyl)amino]methyl}-1,3-thiazole-4-carboxylate (6.7 g) was added to THF (67 mL), and then di-tert-butyl-dicarbonate (4.59 g) was gradually added thereto under ice-cooling, followed by stirring at room temperature for about 14 hours. The reaction mixture was concentrated under reduced pressure and the obtained colorless oily substance was purified by silica gel column chromatography (hexane:ethyl acetate=3:2) to prepare ethyl 2-{[(tert-butoxycarbonyl)(3-phenylpropyl)aminomethyl]methyl}-5-methyl-1,3-thiazole-4-carboxylate (8.34 g).

### Preparation Example 24

Ethyl 2-[([3-(4-fluorophenyl)propyl] {[1-(4-methoxyphenyl)cyclopropyl]carbonyl}amino)methyl]-5-methyl-1,3-thiazole-4-carboxylate (0.25 g) was added to a THF/EtOH (2:1) solution (3 mL), and further, a 1 M aqueous sodium hydroxide solution (1 mL) was added dropwise thereto, followed by stirring at room temperature for about 4 hours. Into the reaction mixture was poured an appropriate amount of a saturated aqueous ammonium chloride solution containing 1 M hydrochloric acid (2.5 mL), and ice water, followed by extraction with ethyl acetate twice. The obtained organic layer was washed with brine and dried over MgSO_{4,} and the solvent was evaporated under reduced pressure to prepare a white residue (0.21 g). This was solidified with a small amount of diethyl ether-diisopropyl ether (1:1), diluted in and washed with the same solvent, and collected by filtration to prepare a white solid of 2-[([3-(4-fluorophenyl)propyl] {[1-(4-methoxyphenyl)cyclopropyl]carbonyl}amino)methyl]-5-methyl-1,3-thiazole-4-carboxylic acid.

### Preparation Example 49

tert-Butyl {[(2-hydroxyethyl)(methyl)amino]sulfonyl}carbamate (1.1 g) and trifluoroacetic acid (2.3 mL) were added to methylene chloride (10 mL), followed by stirring at room temperature for about 15 hours, and then the solvent was evaporated under reduced pressure to prepare a pale yellow oily substance of N-(2-hydroxyethyl)-N-methylsulfamide (0.66 g).

### Preparation Example 51

Ethyl 5-bromo-2-{[{[1-(4-methoxyphenyl)cyclopropyl]carbonyl}(3-phenylpropyl)amino]methyl}-1,3-thiazole-4-carboxylate (415 mg) was added to DMF (5.2 mL), and then Pd (PPh₃)₄(430 mg) and Zn (CN)₂ (110 mg) were sequentially added thereto, followed by stirring at 120°C overnight. The reaction mixture was left to be cooled and then an appropriate amount of ice water was poured thereinto, followed by extraction with ethyl acetate. The organic layer was washed with brine, dried over Na₂SO₄, and evaporated under reduced pressure, and the obtained residue was purified by silica gel column chromatography (hexane to hexane:ethyl acetate=1:1) and repeatedly purified again to prepare a colorless oily substance of ethyl 5-cyano-2-{[{[1-(4-methoxyphenyl)cyclopropyl]carbonyl}(3-phenylpropyl)amino]methyl}-1,3-thiazole-4-carboxylate (65 mg).

### Preparation Example 53

3-Phenylpropan-1-amine (1.46 g) and potassium carbonate (1.64 g) were added to acetonitrile (55 mL), and a solution of ethyl 2-(bromomethyl)-5-chloro-1,3-thiazole-4-carboxylate (2.79 g) in acetonitrile (30 mL) was gradually added dropwise thereto in an ice bath with MeOH, followed by stirring at room temperature for about 4.5 hours. To the reaction mixture was added an appropriate amount of ice water, followed by extraction with ethyl acetate several times. The organic layer was washed with brine and dried over Na₂SO₄, and then the solvent was concentrated under reduced pressure. The obtained residue was purified by silica gel column chromatography (CHCl₃:MeOH=100:0 to 95:5) to prepare ethyl-5-chloro-2-{[(3-phenylpropyl)amino]methyl}-1,3-thiazole-4-carboxylate (3.04 g).

### Preparation Example 72

Under an argon atmosphere, chlorosulfonylisocyanate (0.52 mL) was added to methylene chloride (10 mL), followed by cooling to about -10°C in an MeOH/ice bath. Then, tert-butanol (0.44 g) was added dropwise thereto, followed by stirring for about 30 minutes under cooling. Separately, an appropriate amount of a methylene chloride solution containing triethylamine (1.4 mL) and 2-(methylamino)ethanol (0.4 mL) was prepared and cooled in an ice bath, and the cooled methylene chloride solution was gradually added dropwise thereto, followed by stirring at room temperature for about 30 minutes. Into the reaction mixture was poured an appropriate amount of cooled 0.5 M hydrochloric acid, followed by extraction with an appropriate amount of CHCl₃. The obtained organic layer was dried over MgSO₄ and the solvent was evaporated under reduced pressure to prepare a colorless syrup of tert-butyl {[(2-hydroxyethyl)(methyl)amino]sulfonyl}carbamate (1.15 g).

### Preparation Example 75

Ethyl 2-({[3-(4-fluorophenyl)propyl]amino}methyl)-5-methyl-1,3-thiazole-4-carboxylate (200 mg), 1-(4-methoxyphenyl)cyclopropanecarboxylic acid (130 mg), triethylamine (0.17 mL), and HATU (280 mg) were added to DMF (3 mL), followed by stirring at room temperature for about 15 hours. Into the reaction mixture was poured a cooled aqueous ammonium chloride solution, followed by extraction with an appropriate amount of ethyl acetate. The organic layer was washed sequentially with aqueous sodium bicarbonate and brine, and dried over MgSO_{4.} The solvent was evaporated under reduced pressure and the resulting residue was purified by silica gel column chromatography (hexane:ethyl acetate=3:2) to prepare a yellow oily substance of ethyl 2-[([3-(4-fluorophenyl)propyl]{[1-(4-methoxyphenyl)cyclopropyl]carbonyl}amino)methyl]-5-methyl-1,3-thiazole-4-carboxylate (260 mg).

### Preparation Example 93

[4-(Methylsulfanyl)phenyl]acetonitrile (2.5 g) and N-benzyl-N,N,N-triethylammonium chloride (0.38 g) was added to bromochloroethane (2.8 mL), and a 50% aqueous sodium hydroxide solution (15 mL) was slowly added thereto under ice-cooling. The reaction mixture was stirred at 40°C for 18 hours. To the reaction mixture was added an appropriate amount of ice water, followed by extraction with toluene, and the organic layer was washed with brine, dried over Na₂SO₄, and then evaporated under reduced pressure. The residue was purified by silica gel column chromatography (hexane:ethyl acetate=4:1) to prepare 1-[4-(methylsulfanyl)phenyl]cyclopropanecarbonitrile (2.82 g) as a colorless oily substance.

Next, 1-[4-(methylsulfanyl)phenyl]cyclopropanecarbonitrile (2.82 g) and potassium hydroxide (2.4 g) were added to a mixed solution of purified water (15 mL) and ethylene glycol (15 mL), followed by stirring at 140°C for 4 hours. The reaction mixture was poured into a mixed solution of ice water (100 mL) and 6 M hydrochloric acid (50 mL), and the precipitated solid was collected by filtration and dried under reduced pressure to prepare 1-[4-(methylsulfanyl)phenyl]cyclopropanecarboxylic acid (1.09 g) as a white solid.

### Preparation Example 95

2-Fluoro-4-hydroxybenzaldehyde (1.25 g), triphenylphosphine (3.51 g), and 2,2-difluoroethanol (1.1 g) were added to THF (25 mL), and diisopropyl (E)-diazene-1,2-dicarboxylate (2.7 g) was added thereto under ice-cooling, followed by stirring at room temperature overnight. The reaction mixture was concentrated under reduced pressure and the residue was purified by silica gel column chromatography (hexane to hexane:ethyl acetate=10:1) to prepare a colorless oily substance of 4-(2,2-difluoroethoxy)-2-fluorobenzaldehyde (0.5 g).

### Preparation Example 96

4-Ethoxy-2-fluorobenzaldehyde (1.6 g), triethylamine (0.2 mL), and trimethylsilanecarbonitrile (1.5 mL) were sequentially added to methylene chloride (17 mL), followed by stirring at room temperature overnight. The reaction mixture was concentrated under reduced pressure, and to the residue were added EtOH (16 mL) and chlorotrimethylsilane (3.6 mL), followed by stirring at room temperature overnight. To the reaction mixture was added an appropriate amount of a saturated aqueous sodium hydrogen carbonate solution, followed by stirring at room temperature for 3 hours. The solvent was evaporated under reduced pressure and the resulting residue was extracted with an appropriate amount ethyl acetate, washed with brine, and then dried over Na₂SO₄. The residue was purified by silica gel column chromatography (hexane to hexane:ethyl acetate=2:1) to prepare a white solid of ethyl[4-ethoxy2-fluorophenyl](hydroxy)acetate (1.55 g).

### Preparation Example 99

Ethyl [4-ethoxy-2-fluorophenyl](hydroxy)acetate (1.55 g), [2-(chloromethoxy)ethyl](trimethyl)silane (2.3 mL), a Hunig's base (2.2 mL), and tetra-n-butylammonium iodide (4.73 g) were sequentially added to methylene chloride (15.5 mL), followed by stirring at room temperature overnight. The reaction mixture was concentrated under reduced pressure and an appropriate amount of purified water was added thereto, followed by extraction with ethyl acetate, then washing with brine and drying over Na₂SO₄. The solvent was evaporated under reduced pressure and the residue was purified by silica gel column chromatography (hexane to hexane:ethyl acetate=5:1) to prepare a colorless oily substance of ethyl (4-ethoxy-2-fluorophenyl){[2-(trimethylsily)ethoxy]methoxy}acetate (1.55 g).

### Preparation Example 103

Ethyl 5-methyl-2-{[(3-phenylpropyl)amino]methyl}-1,3-thiazole-4-carboxylate (0.8 g), a Hunig's base (0.15 mL), (4-ethoxy-2-fluorophenyl){[2-(trimethylsilyl)ethoxy]methoxy}acetic acid (0.95 g), and HATU (1.1 g) were sequentially added to acetonitrile (53 mL), followed by stirring at room temperature for 3 hours. The reaction mixture was evaporated under reduced pressure, and to the residue were added an appropriate amount of purified water and 1 M hydrochloric acid, followed by extraction with CHCl₃. The organic layer was dried over Na₂SO₄ and evaporated under reduced pressure. The residue was purified by silica gel column chromatography (hexane to hexane:ethyl acetate=1:1) to prepare ethyl 2-[4-(4-ethoxy-2-fluorophenyl)-10,10-dimethyl-3-oxo-2-(3-phenylpropyl)-5,7-dioxo-2-aza-10-silaneundec-1-yl]-5-methyl-1,3-thiazole-4-carboxylate (1.45 g) as a colorless oily substance.

Next, ethyl 2-[4-(4-ethoxy-2-fluorophenyl)-10,10-dimethyl-3-oxo-2-(3-phenylpropyl)-5,7-dioxo-2-aza-10-silaneundec-1-yl]-5-methyl-1,3-thiazole-4-carboxylate (1.45 g) and a 1 M aqueous sodium hydroxide solution (5 mL) were added to a THF/EtOH (1:1) solution (20 mL), followed by stirring at room temperature overnight. The reaction mixture was evaporated under reduced pressure, and the residue was neutralized by the addition of an appropriate amount of purified water and 1 M hydrochloric acid, and then extracted with ethyl acetate. The organic layer was washed with brine and dried over Na₂SO₄, and then the solvent was evaporated under reduced pressure to prepare 2-[4-(4-ethoxy-2-fluorophenyl)-10,10-dimethyl-3-oxo-2-(3-phenylpropyl)-5,7-dioxo-2-aza-10-silaneundec-1-yl]-5-methyl-1,3-thiazole-4-carboxylic acid (1.35 g).

### Preparation Example 104

1-(4-Hydroxyphenyl)cyclopropanecarboxylic acid (4.5 g) and concentrated sulfuric acid (0.2 mL) were added to EtOH (60 mL), followed by stirring at 70°C for 2 days. The reaction mixture was evaporated under reduced pressure, and to the residue was added an appropriate amount of saturated sodium bicarbonate water, followed by extraction with ethyl acetate. The organic layer was washed with brine and dried over Na₂SO₄, and then the solvent was evaporated under reduced pressure. The obtained residue was purified by silica gel column chromatography (hexane to hexane:ethyl acetate=2:1) to prepare ethyl 1-(4-hydroxyphenyl)cyclopropanecarboxylate (5.0 g) as a pale yellow solid.

### Preparation Example 105

Ethyl 1-(4-hydroxyphenyl)cyclopropanecarboxylate (0.7 g), potassium carbonate (0.7 g), and iodomethane-d₂ were sequentially added to DMF (7 mL), followed by stirring at room temperature overnight. To the reaction mixture was added an appropriate amount of ice water, followed by extraction with ethyl acetate. The organic layer was washed with brine and dried over Na₂SO₄, and the solvent was evaporated under reduced pressure. The obtained residue was purified by silica gel column chromatography (hexane to hexane:ethyl acetate=5:1) to prepare ethyl 1-{4-[(²H₂)methyloxy]phenyl}cyclopropanecarboxylate (0.705 g) as a colorless oily substance.

### Preparation Example 106

N-(2-Hydroxyethyl)-N-methylsulfuric diamide (780 mg), DMAP(309 mg), triethylamine (0.85 mL), and tert-butyl (chloro)dimethylsilane (915 mg) were sequentially added to DMF (8 mL), followed by stirring at room temperature overnight. To the reaction mixture was added an appropriate amount of a saturated aqueous ammonium chloride solution, followed by extraction with CHCl₃, and the organic layer was washed with brine and dried over MgSO₄. The solvent was evaporated under reduced pressure and the obtained residue was purified by silica gel column chromatography (hexane:ethyl acetate=1:1) to prepare N-(2-{[tert-butyl (dimethyl)silyl]oxy}ethyl)-N-methylsulfuric diamide (801 mg) as a white solid.

### Preparation Example 107

Ethyl (2R)-[4-(benzyloxy)phenyl]{[2-(trimethylsilyl)ethoxy]methoxy}acetate (2.65 g), cyclohexene (20 mL), and 10% palladium/carbon (530 mg) were added to EtOH (40 mL), followed by stirring at 100°C for 2 hours. The insoluble material was filtered through Celite, and the obtained filtrate was evaporated under reduced pressure. The obtained residue was purified by silica gel column chromatography (hexane to hexane:ethyl acetate=2:1) to prepare ethyl (2R)-(4-hydroxyphenyl){[2-(trimethylsilyl)ethoxy]methoxy}acetate (2.0 g) as a colorless oily substance.

### Preparation Example 108

Ethyl 2-[(4R)-4-(4-methoxyphenyl)-10,10-dimethyl-3-oxo-2-(3-phenylpropyl)-5,7-dioxa-2-aza-10-silaundec-1-yl]-5-vinyl-1,3-thiazole-4-carboxylate (319 mg) and 10% palladium on carbon (60 mg) were sequentially added to EtOH (40 mL), followed by stirring for about 2 hours at a normal temperature and a normal pressure under a hydrogen gas atmosphere. The catalyst was filtered through celite and the obtained filtrate was evaporated under reduced pressure. To the resulting residue was added an appropriate amount of EtOH again, and 10% palladium on carbon (100 mg) was added thereto, followed by similarly stirring at room temperature for 2 hours under a hydrogen gas atmosphere. This was repeated again (amount of the catalyst: 200 mg) and the residue obtained with the same post-treatment was purified by silica gel column chromatography (hexane to hexane:ethyl acetate=2:1) to prepare a colorless oily substance of ethyl 5-ethyl-2-[(4R)-4-(4-methoxyphenyl)-10,10-dimethyl-3-oxo-2-(3-phenylpropyl)-5,7-dioxa-2-aza-10-silaundec-1-yl]-1,3-thiazole-4-carboxylate (150 mg).

### Preparation Example 109

(2-Fluoro-4-methoxyphenyl)(hydroxy)acetic acid (1.25 g) and (1R)-1-(1-naphthyl)ethanamine (1.07 g) were sequentially added to isopropylalcohol (15 mL), followed by stirring at room temperature for 5 hours. The precipitated solidwas collected by filtration and the resultantwas added to and dissolved in preheated isopropylalcohol (20 mL), and then left to be cooled for recrystallization. The precipitate (0.55 g) obtained through collection by filtration was added to an appropriate amount of purified water, made into a weakly acidic solution with 1 M hydrochloric acid, and extracted with ethyl acetate. The organic layer was washed with brine and then dried over Na₂SO₄, and the solvent was evaporated under reduced pressure to obtain a white solid of (2R)-(2-fluoro-4-methoxyphenyl)(hydroxy)acetic acid (0.28 g).

### Preparation Example 110

(2R)-(2-Fluoro-4-methoxyphenyl)(hydroxy)acetic acid (1.1 g), potassium carbonate (0.9 g), and ethyl iodide (0.6 mL) were sequentially added to DMF (30 mL), followed by stirring at room temperature for 3 hours. To the reaction mixture was added an appropriate amount of purified water, followed by extraction with ethyl acetate. The organic layer was washed with brine and dried over Na₂SO₄, and then the solvent was evaporated under reduced pressure to prepare ethyl (2R)-(2-fluoro-4-methoxyphenyl)(hydroxy)acetate (1.2 g).

Next, ethyl (2R)-(2-fluoro-4-methoxyphenyl)(hydroxy)acetate (270 mg),[2-(chloromethoxy)ethyl](trimethyl) silane (0.42 mL), a Hunig's base (0.42 mL), and tetra-n-butylammonium iodide (440 mg) were sequentially added to methylene chloride (10 mL), followed by stirring at room temperature overnight. The reaction mixture was concentrated under reduced pressure, and the residue was purified by silica gel column chromatography (hexane to hexane:ethyl acetate=5:1) to prepare ethyl(2R)-(2-fluoro-4-methoxyphenyl){[2-(trimethylsilyl)ethoxy]methoxy} acetate (325 mg) as a colorless oily substance.

### Preparation Example 111

Ethyl 5-bromo-2-[(4R)-4-(4-methoxyphenyl)-10,10-dimethyl-3-oxo-2-(3-phenylpropyl)-5,7-dioxo-2-aza-10-silaneundec-1-yl]-1,3-thiazole-4-carboxylate (420 mg), tributyl(vinyl)tin (0.27 mL), (1E,4E)-1,5-diphenylpenta-1,4-dien-3-one_palladium (3:2) (60 mg), and tris(2-methylphenyl)phosphine (75 mg) were sequentially added to toluene (10 mL), followed by stirring at 80°C for 2 hours. The solvent was evaporated under reduced pressure, and the obtained residue was purified by silica gel column chromatography (hexane to hexane:ethyl acetate=1:1) to prepare ethyl 2-[(4R)-4-(4-methoxyphenyl)-10,10-dimethyl-3-oxo-2-(3-phenylpropyl)-5,7-dioxo-2-aza-10-silaneundec-1-yl]-5-vinyl-1,3-thiazole-4-carboxylate (319 mg) as a colorless oily substance.

The compounds of Preparation Examples shown in Tables below were prepared using the respective corresponding starting materials in the same manner as the methods of Preparation Examples above. The structures, the preparation methods, and the physicochemical data for the compounds of Preparation Examples are shown in Tables below.

**[Table 2]**

| Rf | Syn | Structure | Rf | Syn | Structure |
|---|---|---|---|---|---|
| 1 | R1 | 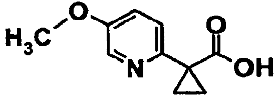 | 2 | R2 | 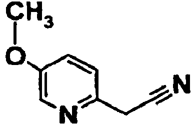 |
| 3 | R3 | 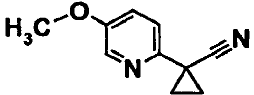 | 4 | R4 | 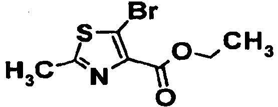 |
| 5 | R5 | 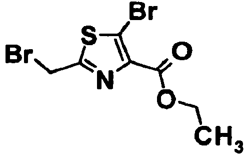 | 6 | R6 | 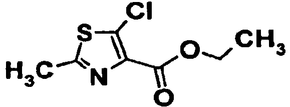 |
| 7 | R7 | 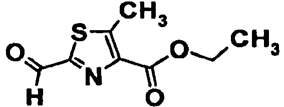 | 8 | R8 | 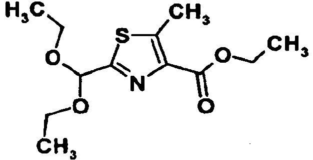 |
| 9 | R9 | 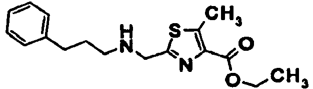 | 10 | R10 | 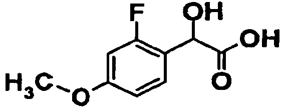 |
| 11 | R11 | 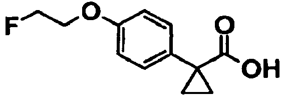 | 12 | R12 | 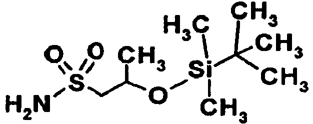 |
| 13 | R13 | 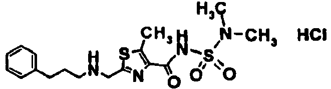 | 14 | R5 | 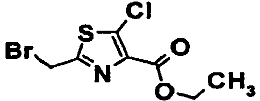 |

**[Table 3]**

| Rf | Syn | Structure | Rf | Syn | Structure |
|---|---|---|---|---|---|
| 17 | R17 | 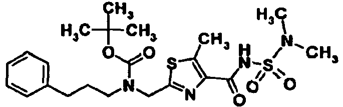 | 18 | R24 | 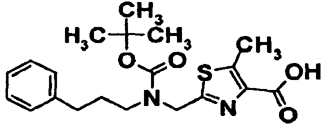 |
| 19 | R19 | 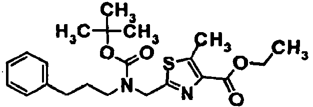 | 22 | R24 | 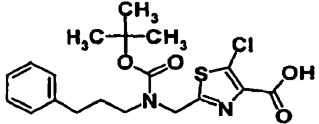 |
| 23 | R24 | 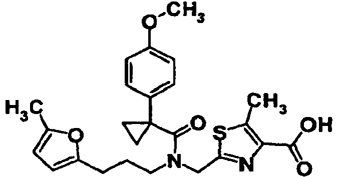 | 24 | R24 | 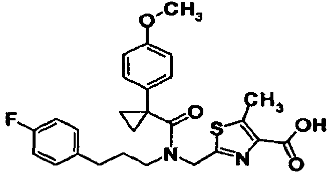 |
| 25 | R24 | 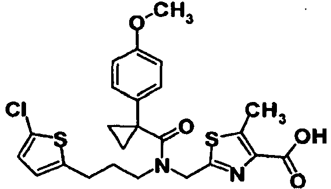 | 26 | R24 | 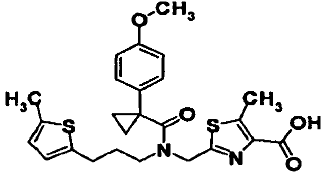 |
| 27 | R24 | 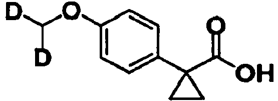 | 28 | R24 | 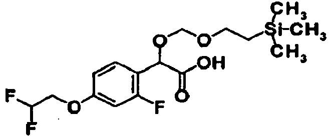 |
| 29 | R24 | 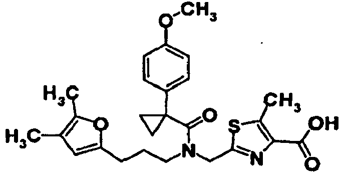 | 30 | R24 | 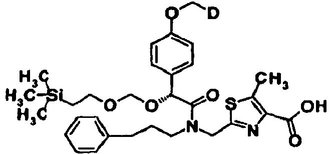 |

**[Table 4]**

| Rf | Syn | Structure | Rf | Syn | Structure |
|---|---|---|---|---|---|
| 31 | R24 | 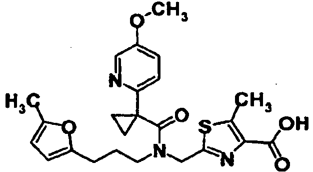 | 32 | R24 | 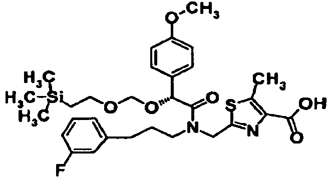 |
| 33 | R24 | 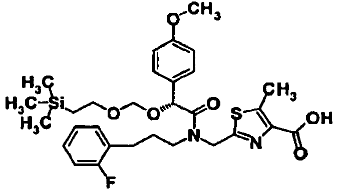 | 34 | R24 | 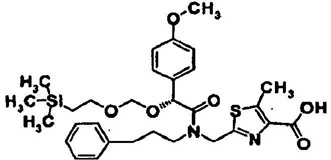 |
| 35 | R24 | 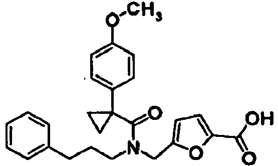 | 36 | R24 | 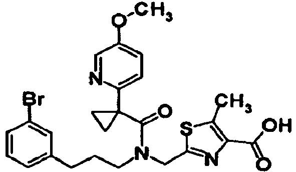 |
| 37 | R24 | 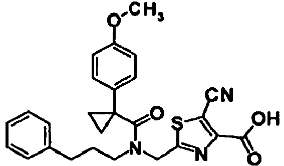 | 38 | R24 | 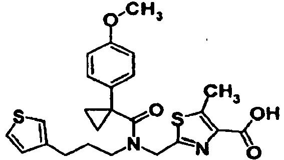 |
| 39 | R24 | 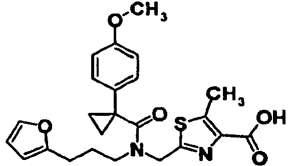 | 40 | R24 | 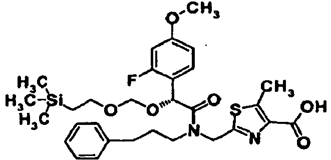 |
| 41 | R24 | 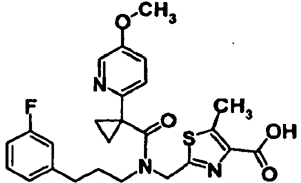 | 42 | R24 | 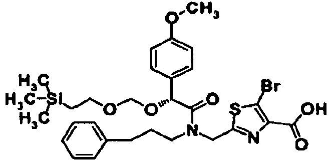 |

**[Table 5]**

| Rf | Syn | Structure | Rf | Syn | Structure |
|---|---|---|---|---|---|
| 43 | R24 | 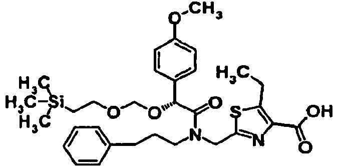 | 45 | R24 | 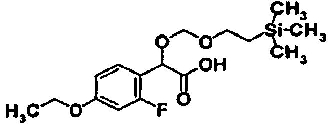 |
| 46 | R24 | 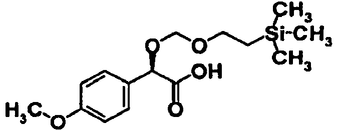 | 47 | R24 | 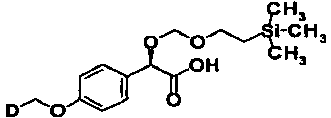 |
| 48 | R24 | 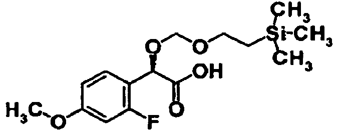 | 49 | R49 | 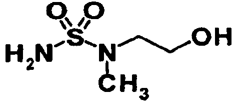 |
| 51 | R51 | 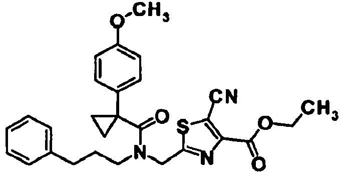 | 52 | R53 | 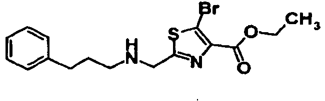 |
| 53 | R53 | 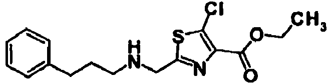 | 54 | R19 | 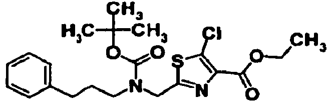 |
| 55 | R13 | 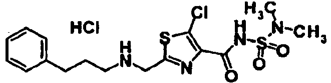 | 56 | R13 | 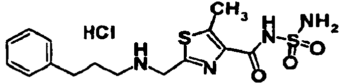 |
| 57 | R17 | 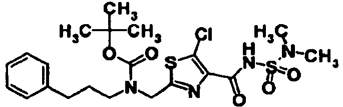 | 58 | R17 | 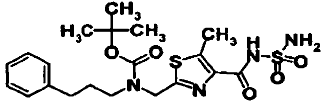 |

**[Table 6]**

| Rf | Syn | Structure | Rf | Syn | Structure |
|---|---|---|---|---|---|
| 59 | R9 | 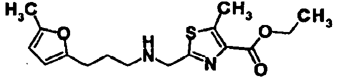 | 60 | R9 | 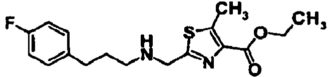 |
| 62 | R9 | 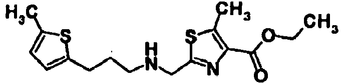 | 63 | R9 | 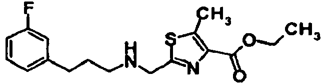 |
| 64 | R9 | 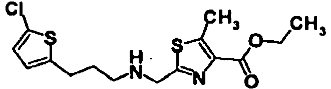 | 65 | R9 | 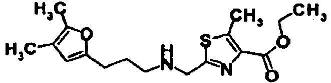 |
| 66 | R9 | 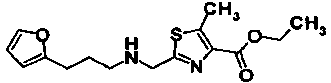 | 67 | R9 | 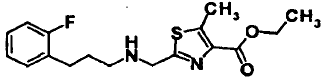 |
| 68 | R9 | 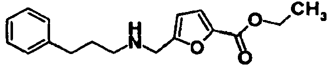 | 70 | R9 | 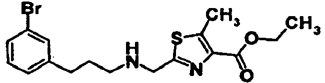 |
| 71 | R9 | 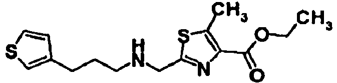 | 72 | R72 | 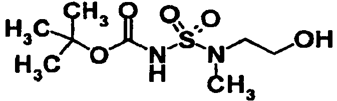 |
| 73 | R75 | 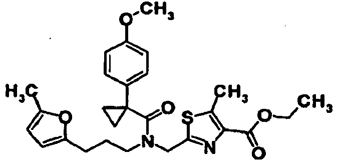 | 74 | R75 | 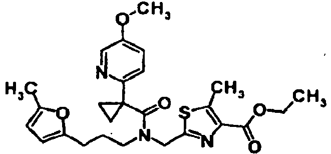 |
| 75 | R75 | 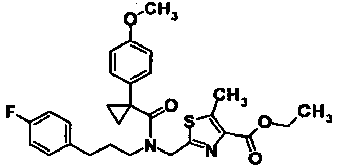 | 76 | R75 | 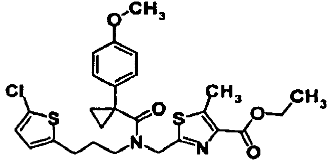 |

**[Table 7]**

| Rf | Syn | Structure | Rf | Syn | Structure |
|---|---|---|---|---|---|
| 77 | R75 | 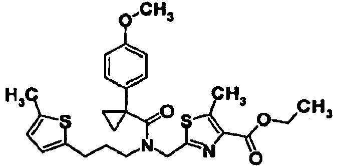 | 78 | R75 | 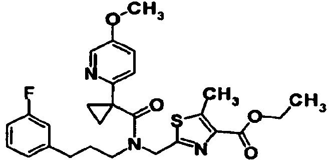 |
| 79 | R75 | 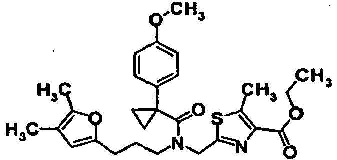 | 80 | R75 | 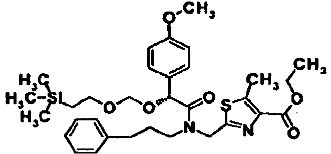 |
| 81 | R75 | 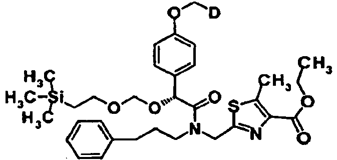 | 82 | R75 | 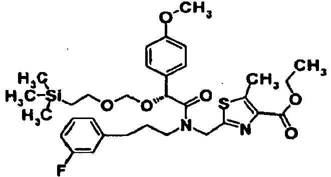 |
| 83 | R75 | 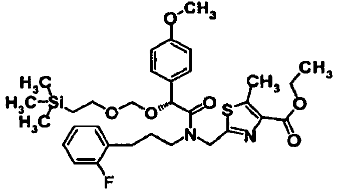 | 84 | R75 | 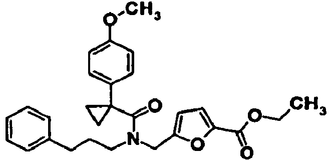 |
| 85 | R75 | 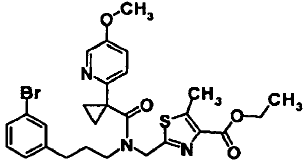 | 86 | R75 | 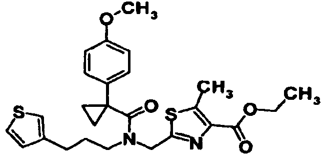 |
| 87 | R75 | 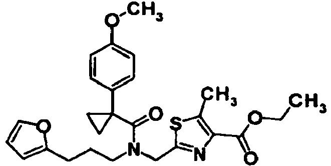 | 88 | R75 | 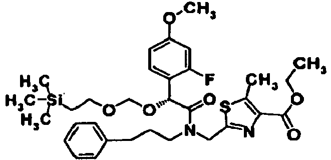 |

**[Table 8]**

| Rf | Syn. | Structure | Rf | Syn | Structure |
|---|---|---|---|---|---|
| 89 | R75 | 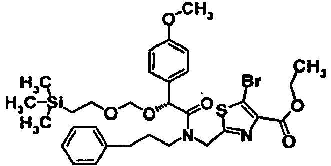 | 90 | R75 | 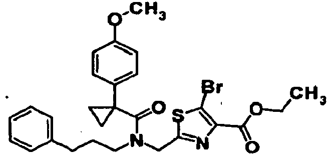 |
| 93 | R93 | 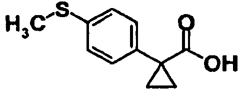 | 94 | R95 | 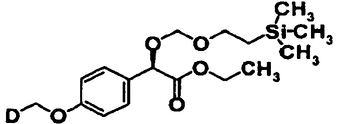 |
| 95 | R95 | 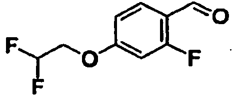 | 96 | R96 | 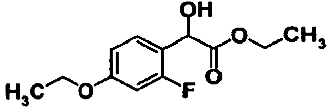 |
| 97 | R96 | 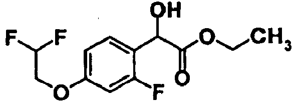 | 98 | R99 | 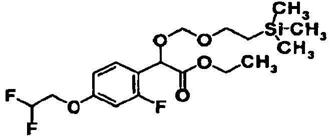 |
| 99 | R99 | 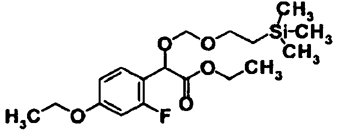 | 100 | R99 | 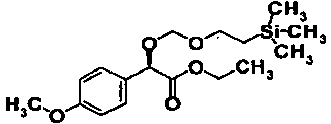 |
| 101 | R99 | 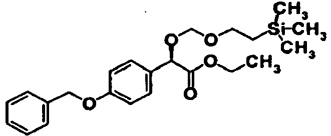 | 102 | R103 | 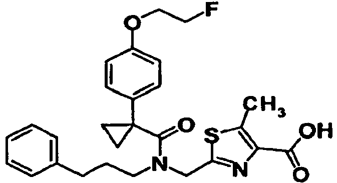 |
| 103 | R103 | 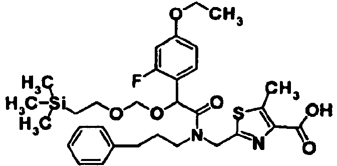 | 104 | R104 | 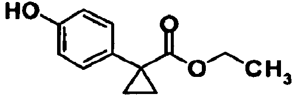 |

**[Table 9]**

| Rf | Syn | Structure | Rf | Syn | Structure |
|---|---|---|---|---|---|
| 105 | R105 | 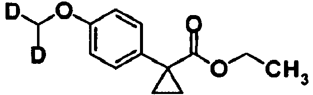 | 106 | R106 | 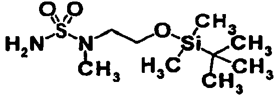 |
| 107 | R107 | 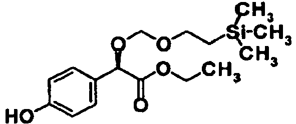 | 108 | R108 | 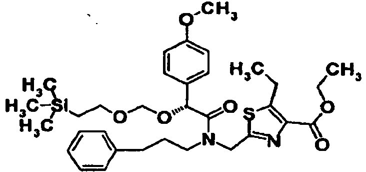 |
| 109 | R109 | 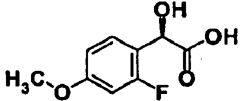 | 110 | R110 | 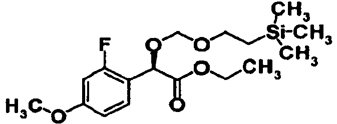 |
| 111 | R111 | 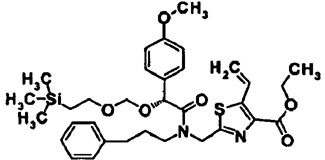 | | | |

**[Table 10]**

| Rf | Data |
|---|---|
| 1 | [ESI+]: 194 |
| 2 | [ESI+]: 149 |
| 3 | [ESI+]: 175 |
| 4 | [EI+]: 249, 251 |
| 5 | [ESI+]: 328, 330, 332 |
| 6 | [ESI+]: 206, 208 |
| 7 | [EI+]: 199 |
| 8 | [FAB+]: 274 |
| 9 | [FAB+]: 319 |
| 10 | [ESI-]:199 |
| 11 | [ESI+]: 225 |
| 12 | [ESI+]: 254 |
| 13 | [ESI+]: 397 |
| 14 | [FAB+]: 285, 287 |
| 17 | [ESI+]: 497 |
| 18 | [ESI+]: 391 |
| 19 | [ESI+]: 419 |
| 22 | [ESI+]: 433, 435 (M+23) |
| 23 | [ESI+]: 469 |
| 24 | [ESI+]: 483 |
| 25 | [ESI+]: 505, 507 |
| 26 | [ESI+]: 485 |
| 27 | [ESI+]: 195 |
| 28 | [ESI-]: 379 |
| 29 | [ESI+]: 483 |
| 30 | [ESI+]: 586 |
| 31 | [ESI+]: 470 |
| 32 | [ESI+]: 603 |
| 33 | [ESI+]: 603 |
| 34 | [ESI+]: 585 |
| 35 | [ESI+]: 434 |
| 36 | [ESI+]: 544, 546 |
| 37 | [APCI+]: 476 |

**[Table 11]**

| Rf | Data |
|---|---|
| 38 | [ESI+]: 471 |
| 39 | [ESI+]: 455 |
| 40 | [FAB-]: 601 |
| 41 | [ESI+]: 484 |
| 42 | [FAB+]: 649, 651 |
| 43 | [ESI+]: 599 |
| 45 | [ESI-]: 343 |
| 46 | [FAB-]: 311 |
| 47 | [FAB-]: 312 |
| 48 | [ESI+]: 353 (M+23) |
| 49 | [APCI+]: 155 |
| 51 | [APCI+]: 504 |
| 52 | [ESI+]: 383, 385 |
| 53 | [ESI+]: 339 |
| 54 | [ESI+]: 461, 463 (M+23) |
| 55 | [EST+]: 417, 419 |
| 56 | [ESI+]: 369 |
| 57 | [ESI+]: 539, 541 (M+23) |
| 58 | [ESI+]: 469 |
| 59 | [ESI+]: 323 |
| 60 | [ESI+]: 337 |
| 62 | [ESI+]: 339 |
| 63 | [ESI+]: 337 |
| 64 | [ESI+]: 359, 361 |
| 65 | [ESI+]: 337 |
| 66 | [ESI+]: 309 |
| 67 | [ESI+]: 337 |
| 68 | [ESI+]: 288 |
| 70 | [ESI+]: 397, 399 |
| 71 | NMR-CDCl₃: 1.41 (3H, t, J = 7.1 Hz), 1.58 (1H, br), 1.84 (2H, m), 3.49 (1H, s), 4.05 (2H, s), 4.41 (2H, q, J = 7.1 Hz), 6.93 (1H, d, J = 4.3 Hz), 7.25 (2H, m). |
| 72 | [EI+]: 254 |
| 73 | [ESI+]: 497 |

**[Table 12]**

| Rf | Data |
|---|---|
| 74 | [ESI+]: 498 |
| 75 | [ESI+]: 511 |
| 76 | [ESI+]: 533, 535 |
| 77 | [ESI+]: 512 |
| 78 | [ESI+]: 512 |
| 79 | [ESI+]: 511 |
| 80 | [ESI+]: 613 |
| 81 | [ESI+]: 614 |
| 82 | [ESI+]: 631 |
| 83 | [ESI+]: 631 |
| 84 | [ESI+]: 462 |
| 85 | [ESI+]: 572, 574 |
| 86 | [ESI+]: 499 |
| 87 | [ESI+]: 483 |
| 88 | [ESI+]: 631 |
| 89 | [ESI+]: 701, 699 (M+23) |
| 90 | [ESI+]: 557, 559 |
| 93 | [ESI+]: 209 |
| 94 | [ESI+]: 364 (M+23) |
| 95 | [ESI+]: 205 |
| 96 | [ESI+]: 265 (M+23) |
| 97 | [ESI+]: 301 (M+23) |
| 98 | [ESI+]: 431 (M+23) |
| 99 | [ESI+]: 395 (M+23) |
| 100 | NMR-CDCl₃: -0.01 (9H, s), 0.87 - 0.92 (2H, m), 1.21 (3H, t, J = 7.1 Hz), 3.54 - 3.73 (2H, m), 3.80 (3H, s), 4.11 - 4.25 (2H, m), 4.68 - 4.78 (2H, m), 5.11 (1H, s), 6.88 (2H, d, J = 8.6Hz), 7.36 (2H, d, J = 8.6Hz). |
| 101 | [ESI+]: 439 (M+23) |
| 102 | [ESI+]: 497 |
| 103 | [ESI-]: 615 |
| 104 | [ESI-]: 205 |
| 105 | [ESI+]: 245 (M+23) |
| 106 | NMR-CDCl₃: 0.10 (6H, s), 0.91 (9H, s), 2.92 (3H, s), 3.39 (2H, t, J = 4.8 Hz), 3.80 (2H, t, J = 4.8 Hz), 4.72 (2H, br). |

**[Table 13]**

| Rf | Data |
|---|---|
| 107 | [FAB-]: 325 |
| 108 | [ESI+]: 627 |
| 109 | [ESI-]: 199 |
| 110 | [ESI+]: 381 (M+23) |
| 111 | [ESI+]: 625 |

### Example 5

2-[([3-(4-Fluorophenyl)propyl]{[1-(4-methoxyphenyl)cyclopropyl]carbonyl}amino)methyl]-5-methyl-1,3-thiazole-4-carboxylic acid (100 mg) and CDI (50 mg) were added to anhydrous THF (4 mL), followed by heating at 60°C for about 1 hour. To the reaction mixture were added N,N-dimethylsulfamide (51 mg) and DBU (38 mg), respectively, under ice-cooling, followed by stirring at room temperature overnight. The reaction mixture was neutralized with an appropriate amount of purified water and 1 M hydrochloric acid, and then extracted with ethyl acetate several times. The obtained organic layer was washed with brine and dried over MgSO₄, and the solvent was evaporated under reduced pressure. The resulting residue was purified by silica gel column chromatography (CHCl₃:MeOH=250:1) and the obtained colorless syrup was solidified with a small amount of ethyl acetate/hexane (1:1) and diethyl ether to prepare a white solid of N-(dimethylsulfamoyl)-2-[([3-(4-fluorophenyl)propyl]{[1-(4-methoxyphenyl)cyclopropyl]carbonyl}amino)methyl]-5-methyl-1, 3-thiazole-4-carboxamide (67 mg).

### Example 13

2-[(4R)-4-(4-Methoxyphenyl)-10,10-dimethyl-3-oxo-2-(3-phenylpropyl)-5,7-dioxa-2-aza-10-silaundec-1-yl]-5-methyl-thiazole-4-carboxylic acid (200 mg) and CDI (83 mg) were added to THF (6 mL), followed by heating at 70°C for about 1 hour. To the reaction mixture were sequentially added sulfamide (66 mg) and DBU (104 mg), followed by stirring at room temperature overnight. To the reaction mixture was added a 4 M hydrochloric acid/dioxane solution (2 mL), followed by stirring at room temperature for 2 hours. The reaction mixture was concentrated under reduced pressure, and to the obtained residue was added purified water, followed by extraction with an appropriate amount of CHCl₃. The organic layer was dried over MgSO₄, the solvent was evaporated under reduced pressure, and the resulting residue was purified by silica gel column chromatography (CHCl₃ to CHCl₃:MeOH=20:1). The purified product was solidified with a small amount of ethyl acetate/hexane (1:1) to prepare a white solid of 2-({[(2R)-2-hydroxy-2-(4-methoxyphenyl)acetyl](3-phenylpropyl)amino}methyl)-5-methyl-N-sulfamoyl-1,3-thiazole-4-carboxamide (138 mg).

### Example 16

5-Chloro-N-(dimethylsulfamoyl)-2-{[(3-phenylpropyl)amino]methyl}-1,3-thiazole-4-carboxamide hydrochloride (1:1) (150 mg), a Hunig's base (0.19 mL), (2R)-(4-methoxyphenyl){[2-(trimethylsilyl)ethoxy]methoxy}acetic acid (114 mg), and HATU (151 mg) were sequentially added to acetonitrile (6 mL), followed by stirring at room temperature overnight. The reaction mixture was evaporated under reduced pressure, the residue was dissolved in THF (6 mL), and a 4 M hydrochloric acid/dioxane solution (3 mL) was added thereto, followed by stirring at room temperature for 2 hours. The reaction mixture was evaporated under reduced pressure, and to the obtained residue was added purified water, followed by extraction with an appropriate amount of CHCl₃. The organic layer was dried over MgSO₄ and the solvent was evaporated under reduced pressure. The resulting residue was purified by silica gel column chromatography (chloroform to chloroform/methanol=20: 1) and the concentrated product was solidified with an appropriate amount of diisopropyl ether to prepare a white solid of 5-chloro-N-(dimethylsulfamoyl)-2-({[(2R)-2-hydroxy-2-(4-methoxyphenyl)acetyl](3-phenylpropyl)amino}methyl)-1,3-thiazole-4-carboxamide (73 mg).

### Example 19

N-[(Dimethylamino)sulfonyl]-2-{[(3-phenylpropyl)amino]methyl}-5-methyl-1,3-thiazole-4-carboxamide hydrochloride (150 mg), (2-cyano-4-methoxyphenyl)acetic acid (80 mg), a Hunig's base (0.2 mL), and HATU (150 mg) were added to THF (10 mL), followed by stirring at room temperature overnight. The reaction mixture was concentrated under reduced pressure and the resulting residue was purified by silica gel column chromatography (hexane to ethyl acetate) and solidified with an appropriate amount of hexane/ethyl acetate (2:1) solution to prepare a white solid of 2-({[(2-cyano-4-methoxyphenyl)acetyl](3-phenylpropyl)amino}methyl)-N-(dimethylsulfamoyl)-5-methyl-1,3-thiazole-4-carboxamide (73 mg).

### Example 20

2-[([3-(3-Bromophenylpropyl]{[1-(5-methoxypyridin-2-yl)cyclopropyl]carbonyl}amino)methyl]-5-methyl-1,3-thiazole-4-carboxylic acid (156 mg) and CDI (67 mg) were added to anhydrous THF (4 mL), followed by heating at 60°C for 1 hour. The reaction mixture was left to be cooled, and then N,N-dimethylsulfamide (68 mg) and DBU (50 mg) were sequentially added thereto, followed by stirring at room temperature overnight. Into the reaction mixture was poured about 15 g of ice water containing 1.5 mL of 1 M hydrochloric acid, followed by extraction with an appropriate amount of ethyl acetate several times, and the obtained organic layer was washed with brine and then dried over MgSO₄. The solvent was evaporated under reduced pressure and the resulting residue was purified by silica gel column chromatography (ethyl acetate/hexane=1:1) to obtain a colorless syrup (120 mg). This was dissolved in a small amount of EtOH, and an appropriate amount of a 4 M hydrochloric acid-ethyl acetate solution was added dropwise thereto under an argon gas atmosphere, followed by stirring for a while. Then, the resulting precipitate was diluted in and washed with diethyl ether/diisopropyl ether (1:1) to prepare a white solid of 2-[([3-(3-bromophenyl)propyl]{[1-(5-methoxypyridin-2-yl)cyclopropyl]carbonyl}amino)methyl]-N-(dimethylsulfamoyl)-5-methyl-1,3-thiazole-4-carboxamide hydrochloride (65 mg).

In the same manner as the methods of Examples above, the Example compounds shown in Tables below were prepared using the respective corresponding starting materials. The structures, the preparation methods, and the physicochemical data of the Example compounds are shown in Tables below.

**[Table 14]**

| Ex | Syn | Structure | Ex | Syn | Structure |
|---|---|---|---|---|---|
| 1 | 13 | 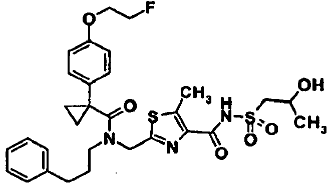 | 2 | 19 | 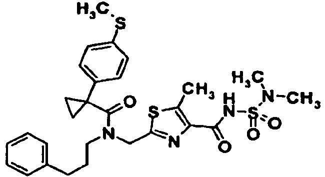 |
| 3 | 5 | 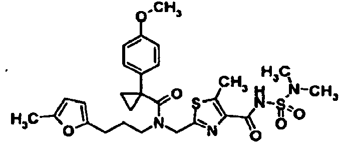 | 4 | 5 | 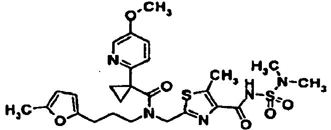 |
| 5 | 5 | 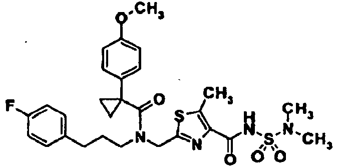 | 6 | 13 | 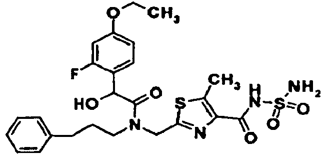 |
| 7 | 5 | 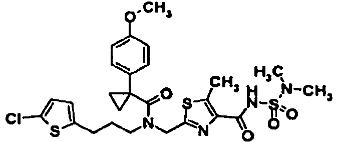 | 8 | 5 | 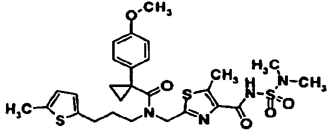 |
| 9 | 19 | 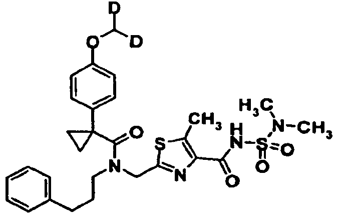 | 10 | 16 | 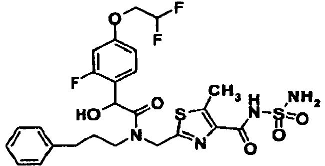 |

**[Table 15]**

| Ex | Syn | Structure | Ex | Syn | Structure |
|---|---|---|---|---|---|
| 11 | 5 | 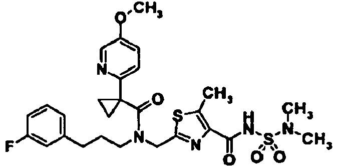 | 12 | 5 | 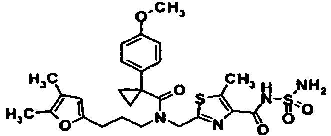 |
| 13 | 13 | 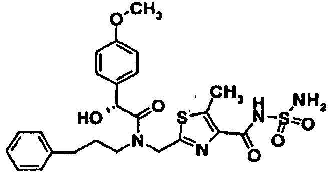 | 14 | 13 | 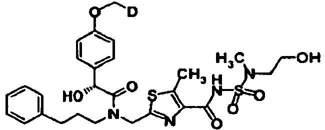 |
| 15 | 13 | 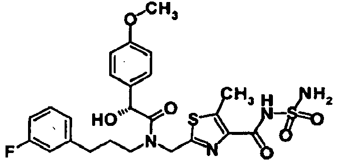 | 16 | 16 | 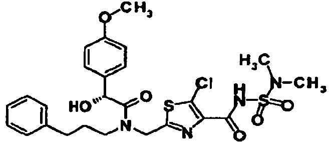 |
| 17 | 5 | 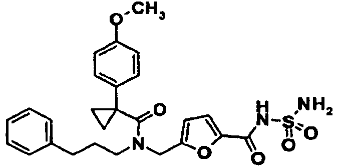 | 18 | 13 | 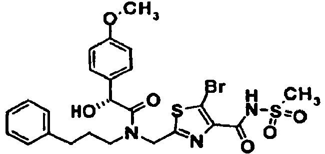 |
| 19 | 19 | 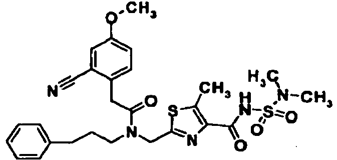 | 20 | 20 | 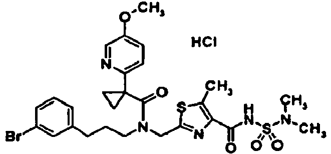 |
| 21 | 5 | 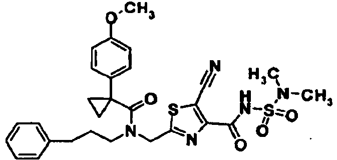 | 22 | 5 | 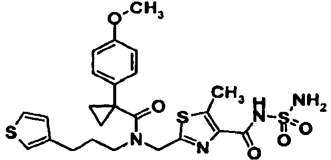 |

**[Table 16]**

| Ex | Syn | Structure | Ex | Syn | Structure |
|---|---|---|---|---|---|
| 23 | 13 | 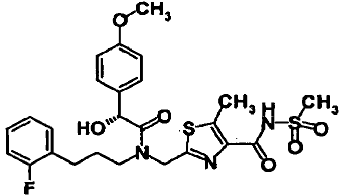 | 24 | 13 | 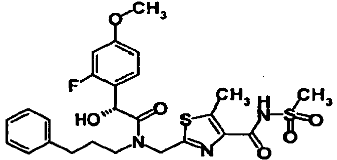 |
| 25 | 13 | 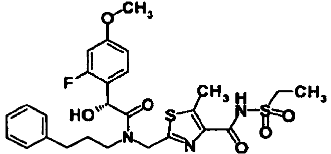 | 26 | 5 | 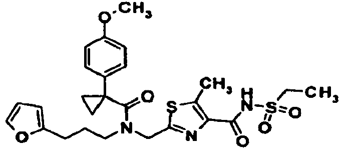 |
| 27 | 13 | 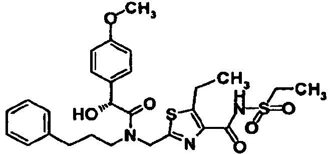 | 28 | 13 | 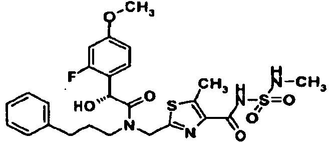 |

**[Table 17]**

| Ex | Data |
|---|---|
| 1 | [ESI+]: 618 |
| | NMR-DMSO-d₆: 1.03 - 1.05 (2H, m), 1.19 - 1.21 (5H, m), 1.31 - 1.35 (2H, m), 2.21 - 2.25 (2H, m), 2.49 - 2.51 (3H, m), 2.6 (1H, br), 2.68 (3H, s), 3.36 (1H, m), 3.46 - 3.51 (1H,m), 3.58-3.64 (1H,m), 4.12-4.15 (2H,m), 4.23 (1H, br), 4.64-4.81 (2H,m), 5.10 (1H, br), 6.91 - 7.26 (9H, m). |
| 2 | [ESI+]: 587 |
| | NMR-DMSO-d₆: 1.08 - 1.39 (6H, m), 2.19 - 2.23 (2H, m), 2.47 (3H, s), 2.67 (3H, s), 2.87 (6H, s), 3.34 - 3.38 (2H, m), 4.68 - 4.78 (2H, m), 6.95 - 7.23 (9H, m), 10.82 (1H, br). |
| 3 | [ESI+]: 575 |
| | NMR-DMSO-d₆: 1.07 (2H, br), 1.24 (2H, br), 1.33 - 1.37 (2H, m), 2.19 (3H, s), 2.21 - 2.27 (2H, m), 2.61 (1H, m), 2.68 (3H, s), 2.88 (6H, s), 3.36 (1H, m), 3.72 (3H, s), 4.68 - 4.80 (2H, m), 5.76 (1H, br), 5.90 (1H, br), 6.87 - 6.89 (2H, m), 7.11 - 7.13 (2H, m), 10.80 (1H, br). |
| 4 | [ESI+]: 576 |
| | NMR-DMSO-d₆: 1.26 - 1.28 (4H, m), 1.41 - 1.48 (2H, m), 2.15 (3H, s), 2.24 (2H, m), 2.63 (1H, m), 2.69 (3H, s), 2.87 (6H, s), 3.35 (1H, m), 3.77 (3H, s), 4.73 - 4.78 (2H, m), 5.72 - 5.95 (2H, m), 7.16 - 7.35 (2H, m), 8.18 (1H, m), 10.9 (1H, br). |
| 5 | [ESI+]: 589 |
| | NMR-DMSO-d₆: 1.05 (2H, br), 1.22 (2H, br), 1.27 - 1.39 (2H, m), 2.18 - 2.25 (2H, m), 2.67 (3H, s), 2.87 (6H, s), 3.27 - 3.39 (2H, m), 3.72 (3H, s), 4.67 (2H, s), 6.78 - 6.90 (2H, m), 6.96 - 7.12 (6H, m), 10.82 (1H, br). |
| 6 | [ESI+]: 565 |
| 7 | [ESI+]: 611 |
| 8 | [ESI+]: 591 |
| | NMR-DMSO-d₆: 1.07 (2H, br), 1.24 (2H, br), 1.32 - 1.36 (2H, m), 2.31 - 2.35 (5H, m), 2.62 (1H, m), 2.67 (3H, s), 2.87 (6H, s), 3.36 (1H, m), 3.72 (3H, s), 4.66 - 4.79 (2H, m), 6.40 (1H, br), 6.54 (1H, br), 6.87 - 6.89 (2H, m), 7.11 - 7.13 (2H, m), 10.80 (1H, br). |
| 9 | [ESI+]: 573 |
| | NMR-DMSO-d₆: 1.00 - 1.39 (6H, m), 2.18 - 2.34 (2H, m), 2.67 (3H, s), 2.87 (6H, s), 3.33 3.40 (2H, m), 3.69 (1H, s), 4.67 - 4.84 (2H, m), 6.87 - 7.25 (9H, m), 10.81 (1H, br). |

**[Table 18]**

| Ex | Data |
|---|---|
| 10 | [ESI+]: 601 |
| | NMR-DMSO-d₆: 1.35 - 1.80 (2H, m), 2.33 - 2.42 (2H, m), 2.68 - 2.69 (3H, m), 3.30 - 3.40 (2H, m), 4.25 - 4.37 (2H, m), 4.66 - 4.91 (2H, m), 5.51 - 5.92 (2H, m), 6.39 (1H, tt, J = 3.5Hz, 54.4Hz), 6.83 - 7.36 (8H, m), 7.59 (2H, br), 10.65 (1H, br). |
| 11 | [ESI+]: 590 |
| | NMR-DMSO-d₆: 1.23 - 1.31 (4H, m), 1.41 - 1.49 (2H, m), 2.20 - 2.28 (2H, m), 2.68 (3H, s), 2.87 (6H, s), 3.31 - 3.39 (2H, m), 3.79 (3H, s), 4.71 - 4.80 (2H, m), 6.73 - 6.80 (2H, m), 6.92 - 7.36 (4H, m), 8.19 (1H, br), 10.85 (1H, br). mp: 114-115 °C |
| 12 | [ESI+]: 561 |
| | NMR-DMSO-d₆: 1.05 (2H, br), 1.23 (2H, br), 1.33 - 1.37 (2H, m), 1.84 (3H, s), 2.09 (3H, s), 2.09 - 2.19 (2H, m), 2.65 (1H, m), 2.70 (3H, s), 3.34 (1H, m), 3.71 (3H, s), 4.66 - 4.77 (2H, m), 5.63 (1H, br), 6.85-7.58 (4H, m), 10.60 (1H, br). |
| 13 | [ESI+]: 533 |
| | NMR-DMSO-d₆: 1.33 - 1.77 (2H, m), 2.29 - 2.40 (2H, m), 2.66 - 2.68 (3H, m), 3.25 - 3.35 (2H, m), 3.73 (3H, s), 4.62 - 4.85 (2H, m), 5.23 - 5.81 (2H, m), 6.86 - 6.91 (2H, m), 7.05 - 7.29 (7H, m), 7.58 - 7.60 (2H, m), 10.66 (1H, br). |
| 14 | [ESI+]: 592 |
| | NMR-DMSO-d₆: 1.32 - 1.76 (2H, m), 2.28 - 2.40 (2H, m), 2.63 - 2.65 (3H, m), 2.90 - 2.92 (3H, m), 3.27 - 3.35 (4H, m), 3.54 - 3.60 (2H, m), 3.70 - 3.73 (2H, m), 4.62 - 4.86 (2H, m), 4.97 (1H, br), 5.24 - 5.84 (2H, m), 6.86 - 6.91 (2H, m), 7.04 - 7.29 (7H, m), 10.96 (1H, br). |
| 15 | [ESI+]: 551 |
| | NMR-DMSO-d₆: 1.32 - 1.78 (2H, m), 2.32 - 2.41 (2H, m), 2.66 - 2.68 (3H, m), 3.25 - 3.33 (2H, m), 3.72 (3H, s), 4.62 - 4.86 (2H, m), 5.25 - 5.81 (2H, m), 6.85 - 7.33 (8H, m), 7.57 (2H, br), 10.65 (1H, br). |
| 16 | [ESI+]: 581,583 |
| 17 | [ESI+]: 512 |
| | NMR-DMSO-d₆: 0.97 - 1.37 (6H, m), 2.24 - 2.26 (2H, m), 2.49 - 2.50 (2H, m), 3.20 - 3.25 (2H, m), 3.72 (3H, s), 4.55 (2H, br), 6.19 - 6.32 (2H, m), 6.85 - 7.51 (9H, m), 11.7 (1H, br). |
| 18 | [ESI+]: 596, 598 |

**[Table 19]**

| Ex | Data |
|---|---|
| 19 | [ESI+]: 570 |
| | NMR-DMSO-d₆: 1.77 - 1.99 (2H, m), 2.52 - 2.71 (5H, m), 2.86 (6H, s), 3.40 - 3.58 (2H, m), 3.80 - 3.81 (3H, m), 3.90 - 3.97 (2H, m), 4.72 - 4.99 (2H, m), 7.15 - 7.40 (8H, m), 10.83 (1H, br). |
| 20 | [ESI+]: 650, 652 |
| 21 | [ESI+]: 580 |
| 22 | [ESI+]: 549 |
| | NMR-DMSO-d₆: 1.04 - 1.36 (6H, m), 2.24 - 2.31 (2H, m), 2.70 (3H, s), 3.29 - 3.35 (2H, m), 3.71 (3H, s), 4.66 - 4.77 (2H, m), 6.79 - 7.09 (4H, m), 7.42 - 7.58 (3H, m), 11.6 (1H, br). |
| 23 | [ESI+]: 550 |
| 24 | [ESI+]: 550 |
| | NMR-DMSO-d₆: 1.22 - 1.81 (2H, m), 2.33 - 2.40 (2H, m), 2.66 - 2.68 (3H, m), 3.33 - 3.41 (5H, m), 3.74 - 3.76 (3H, m), 4.66 - 4.89 (2H, m), 5.50 - 5.86 (2H, m), 6.71 - 6.85 (2H, m), 7.05 - 7.31 (6H, m), 11.11 (1H, br). mp: 121-123 °C |
| 25 | [ESI+]: 564 |
| | NMR-DMSO-d₆: 1.24 (3H, t, J = 7.3Hz), 1.32 - 1.80 (2H, m), 2.32 - 2.40 (2H, m), 2.65 - 2.67 (3H, m), 3.32 - 3.50 (4H, m), 3.74 - 3.76 (3H, m), 4.66 - 4.90 (2H, m), 5.50 - 5.86 (2H, m), 6.71 - 6.85 (2H, m), 7.04 - 7.33 (6H, m), 11.10 (1H, br). mp: 111-112 °C |
| 26 | [ESI+]: 546 |
| 27 | [ESI+]: 560 |
| | NMR-DMSO-d₆: 1.15 - 1.27 (6H, m), 1.38 - 1.76 (2H, m), 2.30 - 2.43 (2H, m), 3.08 - 3.17 (2H, m), 3.32 - 3.50 (4H, m), 3.71 - 3.73 (3H, m), 4.62 - 4.88 (2H, m), 5.24 - 5.82 (2H, m), 6.89 - 7.29 (9H, m), 11.11 (1H, br). |
| 28 | [ESI+]: 565 |
| | NMR-DMSO-d₆: 1.33 - 1.82 (2H, m), 2.32 - 2.42 (2H, m), 2.53 (3H, s), 2.68 (3H, s), 3.34 - 3.42 (2H, m), 3.74 - 3.76 (3H, m), 4.64 - 4.92 (2H, m), 5.50 - 5.87 (2H, m), 6.69 - 6.86 (2H, m), 7.02 - 7.3 2 (6H, m), 7.62 (1H, br), 10.71 (1H, br). |

The compound of the present invention or salts thereof have an excellent LPA receptor antagonistic action and can be used for preventing and/or treating diseases caused by LPA.

## Claims

1. The compound 2-({[(2-cyano-4-methoxyphenyl)acetyl](3-phenylpropyl)amino}methyl)-N-(dimethylsulfamoyl)-5-methyl-1,3-thiazole-4-carboxamide, or a salt thereof.

2. A pharmaceutical composition comprising the compound or a salt thereof according to Claim 1 and a pharmaceutically acceptable excipient.

3. The compound or a salt thereof according to Claim 1 for use in a method for preventing and/or treating diseases caused by LPA selected from the group consisting of benign prostatic hyperplasia, urinary dysfunction associated with benign prostatic hyperplasia, bladder neck sclerosis and underactive bladder.

4. The compound or a salt thereof according to Claim 1 for use in a method for preventing and/or treating diseases caused by LPA selected from the group consisting of breast cancer, chronic renal diseases associated with fibrosis, and idiopathic pulmonary fibrosis.

## Patentansprüche

1. Verbindung 2-({(2-Cyano-4-methoxyphenyl)acetyl](3-phenylpropyl)amino}methyl)-N-(dimethylsulfamoyl)-5-methyl-1,3-thiazol-4-carboxamid oder ein Salz davon.

2. Pharmazeutische Zusammensetzung, die eine Verbindung oder ein Salz davon gemäss Anspruch 1 und einen pharmazeutisch annehmbaren Hilfsstoff umfasst.

3. Verbindung oder ein Salz davon gemäss Anspruch 1 zur Verwendung in einem Verfahren zur Prävention und/oder Behandlung von Erkrankungen, die durch LPA verursacht werden, ausgewählt aus der Gruppe bestehend aus benigner Prostatahyperplasie, Harnwegsdysfunktion in Verbindung mit benigner Prostatahyperplasie, Blasenhalssklerose und einer Unterfunktion der Blase.

4. Verbindung oder ein Salz davon gemäss Anspruch 1 zur Verwendung in einem Verfahren zur Prävention und/oder Behandlung von Erkrankungen, die durch LPA verursacht werden, ausgewählt aus der Gruppe bestehend aus Brustkrebs, chronischen Nierenerkrankungen in Verbindung mit Fibrose und idiopathischer Lungenfibrose.

## Revendications

1. Composé 2-({[(2-cyano-4-méthoxyphényl)acétyl](3-phénylpropyl)amino}méthyl)-N-{diméthylsulfamoyl)-5-méthyl-1,3-thiazole-4-carboxamide, ou un sel de celui-ci.

2. Composition pharmaceutique comprenant le composé ou un sel de celui-ci selon la revendication 1 et un excipient pharmaceutiquement acceptable.

3. Composé ou un sel de celui-ci selon la revendication 1 pour utilisation dans un procédé de prévention et/ou traitement de maladies causées par LPA choisies dans le groupe constitué d'une hyperplasie bégnine de la prostate, un dysfonctionnement urinaire associé à une hyperplasie bégnine de la prostate, une sclérose du col vésical et la vessie paresseuse.

4. Composé ou un sel de celui-ci selon la revendication 1 pour utilisation dans un procédé de prévention et/ou traitement de maladies causées par LPA choisies dans le groupe constitué du cancer du sein, de maladies rénales chroniques associées à une fibrose, et d'une fibrose pulmonaire idiopathique.
